# EUROPEAN PATENT APPLICATION

(11) **EP 4 299 593 A1**
(43) Date of publication of application: **03.01.2024**
(21) Application number: 22779155.5
(22) Date of filing: 01.04.2022
(51) Int. Cl.: C07K 16/28, C07K 16/30, A61K 39/395, A61P 35/00, C12N 15/13, C12N 15/63

(54) **CLDN18.2 ANTIGEN-BINDING PROTEIN AND USE THEREOF**

(30) Priority: 02.04.2021 CN 202110359711; 07.01.2022 CN 202210016145
(71) Applicant: Oricell Therapeutics Co., Ltd., Shanghai 201203 (CN)
(72) Inventor: YANG, Shasha, Shanghai 201203 (CN); HE, Xiaowen, Shanghai 201203 (CN); LI, Meng, Shanghai 201203 (CN); WANG, Huajing, Shanghai 201203 (CN); YANG, Huanfeng, Shanghai 201203 (CN)
(74) Representative: Weickmann & Weickmann PartmbB
(86) International application number: PCT/CN2022/084922
(87) International publication number: WO 2022/206975

(57) **Abstract**

Provided is an isolated antigen-binding protein, capable of binding to CLDN18.2. Also provided are a chimeric antigen receptor comprising the isolated antigen binding protein, and a preparation method for and an application of the antigen binding protein and the chimeric antigen receptor.

## Description

### FIELD OF THE INVENTION

This application relates to the field of biomedicine, and in particular to an antigen-binding protein binding to CLDN18.2.

### BACKGROUND OF THE INVENTION

Gastric cancer is the second most lethal malignant tumor in the world. According to 2015 data from the World Health Organization, 754,000 gastric cancer patients die every year worldwide. Pancreatic cancer is one of the most malignant tumors. According to data from The Lancet in 2016, more than 200,000 pancreatic cancer patients die every year worldwide. The standard first-line treatment for advanced or recurrent gastric cancer is chemotherapy. The addition of trastuzumab to chemotherapy provides some survival benefit for patients with HER2-positive tumors, but only 15% of all gastric cancer patients are HER2-positive. It is urgent to develop safe and effective treatments.

CLDN18.2 (Claudin18.2) is only expressed in differentiated gastric parietal cells and is not expressed in normal tissues. The latest research shows that CLDN18.2 is overexpressed in more than 77% of gastric cancer patients and more than 80% of pancreatic cancer patients. In addition, it is overexpressed in solid tumors such as lung cancer, esophageal cancer, ovarian cancer, etc. CLDN18.2 belongs to a family of tight junction proteins that control the flow of molecules between lamellar cells. However, in tumors, the tight junctions are disrupted and the CLDN protein loses its primary role. Because CLDN18.2 is abundantly present in gastric tumors, the researchers estimated that half of all patients with advanced gastric cancer could be candidates for new therapies targeting CLDN18.2 antibodies. Additionally, this unique target is absent from any healthy tissue other than the stomach lining, thereby minimizing treatment side effects. These features suggest that CLDN18.2 is an ideal target for development of therapeutic monoclonal antibodies.

Since CLDN18.2 is a membrane protein, it is difficult to obtain high-quality antibodies against natural proteins using traditional antibody screening methods. At present, in vivo immunization is mostly used, such as the method of injecting DNA, but multiple rounds of immunization on animals are required. CLDN18 has two splicing variants, CLDN18.1 and CLDN18.2. The two variants differ only in 69 amino acid sequences at the N terminal, which are present in the first extracellular loop, and the rest of the sequences are identical. CLDN18.1 is expressed in normal lung tissue, so it is necessary to screen for antibodies that can bind to CLDN18.2 but not bind to CLDN18.1. On the other hand, the sequence of CLDN18.2 is highly homologous between human and murine. Therefore, there is an urgent need to obtain antibodies that specifically bind to CLDN18.2.

### SUMMARY OF THE INVENTION

This application provides an antigen-binding protein binding to CLDN18.2, which exhibits one or more desired functional properties. For example, it can specifically bind to human CLDN18.2 and essentially does not bind to human CLDN18.1, and can have CDC activity and/or have anti-tumor activity. This application further provides a nucleic acid molecule encoding the isolated antigen-binding protein, an expression vector, a host cell and a method for preparing the isolated antigen-binding protein. The isolated antigen-binding protein of this application can be used for the prevention and/or treatment of a disease and/or a disorder, such as a tumor and/or a cancer.

In one aspect, this application provides an isolated antigen-binding protein comprising an HCDR3, wherein said HCDR3 comprises an amino acid sequence as set forth in SEQ ID NO: 67.

In certain embodiments, said HCDR3 of the isolated antigen-binding protein comprises an amino acid sequence as set forth in any one of SEQ ID NO: 4, SEQ ID NO: 12, and SEQ ID NO: 18.

In certain embodiments, the isolated antigen-binding protein comprises an HCDR2, wherein said HCDR2 comprises an amino acid sequence as set forth in SEQ ID NO: 68 or SEQ ID NO: 11.

In certain embodiments, said HCDR2 of the isolated antigen-binding protein comprises an amino acid sequence as set forth in SEQ ID NO: 3 or SEQ ID NO: 17.

In certain embodiments, the isolated antigen-binding protein comprises an HCDR1, wherein said HCDR1 comprises an amino acid sequence as set forth in any one of SEQ ID NO: 2, SEQ ID NO: 10, and SEQ ID NO: 16.

In certain embodiments, the isolated antigen-binding protein comprises an H-FR1, a C terminal of said H-FR1 is directly or indirectly linked to an N terminal of said HCDR1, and said H-FR1 comprises an amino acid sequence as set forth in SEQ ID NO: 72.

In certain embodiments, said H-FR1 of the isolated antigen-binding protein comprises an amino acid sequence as set forth in any one of SEQ ID NO: 34, SEQ ID NO: 43, and SEQ ID NO: 49.

In certain embodiments, the isolated antigen-binding protein comprises an H-FR2, wherein said H-FR2 is located between the HCDR1 and the HCDR2 and comprises an amino acid sequence as set forth in SEQ ID NO: 73.

In certain embodiments, said H-FR2 of the isolated antigen-binding protein comprises an amino acid sequence as set forth in SEQ ID NO: 35 or SEQ ID NO: 44.

In certain embodiments, the isolated antigen-binding protein comprises an H-FR3, wherein said H-FR3 is located between the HCDR2 and the HCDR3 and comprises an amino acid sequence as set forth in SEQ ID NO: 80 or SEQ ID NO: 45.

In certain embodiments, said H-FR3 of the isolated antigen-binding protein comprises an amino acid sequence as set forth in SEQ ID NO: 36 or SEQ ID NO: 42.

In certain embodiments, the isolated antigen-binding protein comprises an H-FR4, an N terminal of said H-FR4 is linked to a C terminal of said HCDR3, and the H-FR4 comprises an amino acid sequence as set forth in SEQ ID NO: 74.

In certain embodiments, said H-FR4 of the isolated antigen-binding protein comprises an amino acid sequence as set forth in SEQ ID NO: 46 or SEQ ID NO: 37.

In certain embodiments, the isolated antigen-binding protein comprises a VH, where said VH comprises an amino acid sequence as set forth in SEQ ID NO: 78.

In certain embodiments, said VH of the isolated antigen-binding protein comprises an amino acid sequence as set forth in any one of SEQ ID NO: 1, SEQ ID NO: 9, SEQ ID NO: 15, and SEQ ID NO: 19.

In certain embodiments, the isolated antigen-binding protein comprises an LCDR3, wherein said LCDR3 comprises an amino acid sequence as set forth in SEQ ID NO: 69.

In certain embodiments, said LCDR3 of the isolated antigen-binding protein comprises an amino acid sequence as set forth in any one of SEQ ID NO: 8, SEQ ID NO: 14, SEQ ID NO: 23, and SEQ ID NO: 25.

In certain embodiments, the isolated antigen-binding protein comprises an LCDR2, wherein said LCDR2 comprises an amino acid sequence as set forth in SEQ ID NO: 70.

In certain embodiments, said LCDR2 of the isolated antigen-binding protein comprises an amino acid sequence as set forth in SEQ ID NO: 7 or SEQ ID NO: 22.

In certain embodiments, the isolated antigen-binding protein comprises an LCDR1, wherein said LCDR1 comprises an amino acid sequence as set forth in SEQ ID NO: 71.

In certain embodiments, said LCDR1 of the isolated antigen-binding protein comprises an amino acid sequence as set forth in SEQ ID NO: 6 or SEQ ID NO: 21.

In certain embodiments, the isolated antigen-binding protein comprises an L-FR1, a C terminal of said L-FR1 is directly or indirectly linked to an N terminal of said LCDR1, and the L-FR1 comprises an amino acid sequence as set forth in SEQ ID NO: 75.

In certain embodiments, said L-FR1 of the isolated antigen-binding protein comprises an amino acid sequence as set forth in any one of SEQ ID NO: 38, SEQ ID NO: 47, SEQ ID NO: 50 and SEQ ID NO: 53.

In certain embodiments, the isolated antigen-binding protein comprises an L-FR2, wherein said L-FR2 is located between said LCDR1 and said LCDR2 and comprises an amino acid sequence as set forth in SEQ ID NO: 39.

In certain embodiments, the isolated antigen-binding protein comprises an L-FR3, and said L-FR3 is located between said LCDR2 and said LCDR3 and comprises an amino acid sequence as set forth in SEQ ID NO: 76.

In certain embodiments, said L-FR3 of the isolated antigen-binding protein comprises an amino acid sequence as set forth in any one of SEQ ID NO: 40, SEQ ID NO: 48, and SEQ ID NO: 51.

In certain embodiments, the isolated antigen-binding protein comprises an L-FR4, an N terminal of said L-FR4 is linked to a C terminal of the LCDR3, and said L-FR4 comprises an amino acid sequence as set forth in SEQ ID NO: 77.

In certain embodiments, said L-FR4 of the isolated antigen-binding protein comprises an amino acid sequence as set forth in any one of SEQ ID NO: 41, SEQ ID NO: 52, and SEQ ID NO: 54.

In certain embodiments, the isolated antigen-binding protein comprises a VL, wherein said VL comprises an amino acid sequence as set forth in SEQ ID NO: 79.

In certain embodiments, said VL of the isolated antigen-binding protein comprises an amino acid sequence as set forth in any one of SEQ ID NO: 5, SEQ ID NO: 13, SEQ ID NO: 20, and SEQ ID NO: 24.

In certain embodiments, the isolated antigen-binding protein comprises any set of amino acid sequences selected from the group consisting of:
1) said VH comprising the amino acid sequence as set forth in SEQ ID NO: 1 and said VL comprising the amino acid sequence as set forth in SEQ ID NO: 5;
2) said VH comprising the amino acid sequence as set forth in SEQ ID NO: 15 and said VL comprising the amino acid sequence as set forth in SEQ ID NO: 5;
3) said VH comprising the amino acid sequence as set forth in SEQ ID NO: 9 and said VL comprising the amino acid sequence as set forth in SEQ ID NO: 13;
4) said VH comprising the amino acid sequence as set forth in SEQ ID NO: 19 and the VL comprising the amino acid sequence as set forth in SEQ ID NO: 20; and
5) said VH comprising the amino acid sequence as set forth in SEQ ID NO: 19 and the VL comprising the amino acid sequence as set forth in SEQ ID NO: 24.

In certain embodiments, the isolated antigen-binding protein comprises an antibody heavy chain constant region.

In certain embodiments, said antibody heavy chain constant region is derived from a human IgG heavy chain constant region.

In certain embodiments, said antibody heavy chain constant region is derived from a human IgG1 heavy chain constant region.

In certain embodiments, the isolated antigen-binding protein comprises an antibody light chain constant region.

In certain embodiments, said antibody light chain constant region is derived from a human Igκ constant region.

In certain embodiments, the isolated antigen-binding protein comprises an antibody or an antigen-binding fragment thereof.

In certain embodiments, said antigen-binding fragment includes Fab, Fab', Fv fragment, F(ab')₂, F(ab)₂, scFv, di-scFv and/or dAb.

In certain embodiments, said antibody is selected from one or more groups consisting of a monoclonal antibody, a chimeric antibody, a humanized antibody, and a fully human antibody.

In certain embodiments, said isolated antigen binding protein does not substantially compete with a reference antibody for binding to CLDN18.2, as verified in FACS, wherein said reference antibody comprises a heavy chain variable region (VH) and a light chain variable region (VL), the VH of said reference antibody comprises an amino acid sequence as set forth in SEQ ID NO: 55, and the VL of said reference antibody comprises an amino acid sequence as set forth in SEQ ID NO: 56.

In certain embodiments, said isolated antigen-binding protein specifically binds to CLDN18.2 and does not substantially bind to CLDN18.1.

In certain embodiments, said CLDN18.2 to which the isolated antigen binding protein specifically binds includes mouse CLDN18.2, cynomolgus monkey CLDN18.2 and/or human CLDN18.2.

In certain embodiments, said isolated antigen-binding protein has CDC activity.

In certain embodiments, said isolated antigen-binding protein is capable of inhibiting tumor growth and/or proliferation of tumor cells.

In another aspect, the present application further provides a chimeric antigen receptor comprising a targeting moiety, wherein said targeting moiety comprises said antigen-binding protein described in the present application.

In certain embodiments, said chimeric antigen receptor comprises a costimulatory domain, wherein said costimulatory domain comprises a costimulatory domain derived from one or more proteins selected from the group consisting of CD28, 4-1BB, CD27, CD2, CD7, CD8, OX40, CD226, DR3, SLAM, CDS, ICAM-1, NKG2D, NKG2C, B7-H3, 2B4, FcεRIγ, BTLA, GITR, HVEM, DAP10, DAP12, CD30, CD40, CD40L, TIM1, PD-1, LFA-1, LIGHT, JAML, CD244, CD100, ICOS, a ligand of CD83, CD40, and MyD88.

In certain embodiments, said costimulatory domain of the chimeric antigen receptor is an intracellular costimulatory signaling domain derived from 4-1BB.

In certain embodiments, said costimulatory domain of the chimeric antigen receptor comprises an amino acid sequence as set forth in SEQ ID NO: 85.

In certain embodiments, said chimeric antigen receptor comprises an intracellular signaling domain, wherein said intracellular signaling domain comprises an intracellular signaling domain derived from one or more proteins selected from the group consisting of CD3ζ, CD3δ, CD3γ, CD3ε, CD79a, CD79b, FcεRIγ, FcεRIβ, FcyRIIa, bovine leukemia virus gp30, Epstein-Barr virus (EBV) LMP2A, simian immunodeficiency virus PBj 14Nef, Kaposi's Sarcoma Associated-Herpesvirus (HSKV), DAP10, DAP-12, and a domain containing at least one ITAM.

In certain embodiments, said intracellular signaling domain of the chimeric antigen receptor is a signaling domain derived from CD3ζ.

In certain embodiments, said intracellular signaling domain of the chimeric antigen receptor comprises an amino acid sequence as set forth in SEQ ID NO: 86.

In certain embodiments, said chimeric antigen receptor comprises a transmembrane domain, wherein said transmembrane domain comprises a transmembrane domain derived from one or more proteins selected from the group consisting of: CD8, CD28, 4-1BB, CD4, CD27, CD7, PD-1, TRAC, TRBC, CD3ε, CD3ζ, CTLA-4, LAG-3, CD5, ICOS, OX40, NKG2D, 2B4, CD244, FcεRIγ, BTLA, CD30, GITR, HVEM, DAP10, CD2, NKG2C, LIGHT, DAP12, CD40L, TIM1, CD226, DR3, CD45, CD80, CD86, CD9, CD16, CD22, CD33, CD37, CD64, CD134, CD137, CD154, and SLAM.

In certain embodiments, said transmembrane domain of the chimeric antigen receptor is a transmembrane domain derived from CD8.

In certain embodiments, said transmembrane domain of the chimeric antigen receptor comprises an amino acid sequence as set forth in SEQ ID NO: 84.

In certain embodiments, said chimeric antigen receptor comprises a hinge region between the targeting moiety and the transmembrane domain, wherein said hinge region comprises a hinge region derived from one or more proteins selected from the group consisting of CD28, IgG1, IgG4, IgD, 4-1BB, CD4, CD27, CD7, CD8, PD-1, ICOS, OX40, NKG2D, NKG2C, FcεRIγ, BTLA, GITR, DAP10, CD40L, TIM1, CD226, SLAM, CD30, and LIGHT.

In certain embodiments, said hinge region of the chimeric antigen receptor is a hinge region derived from CD8.

In certain embodiments, said hinge region of the chimeric antigen receptor comprises an amino acid sequence as set forth in SEQ ID NO: 83.

In certain embodiments, said chimeric antigen receptor further comprises a signal peptide.

In certain embodiments, said signal peptide of the chimeric antigen receptor is a signal peptide derived from the CD8 protein.

In certain embodiments, said signal peptide of the chimeric antigen receptor comprises an amino acid sequence as set forth in SEQ ID NO: 88.

In certain embodiments, said chimeric antigen receptor further comprises a low-density lipoprotein receptor-related protein or a fragment thereof.

In certain embodiments, said low-density lipoprotein receptor-related protein or the fragment thereof comprises one or more groups selected from: low-density lipoprotein receptor-related proteins 1-12 and functional fragments thereof.

In certain embodiments, said low-density lipoprotein receptor-related protein or the fragment thereof comprises an amino acid sequence as set forth in SEQ ID NO: 91.

In another aspect, the present application further provides a polypeptide molecule comprising said isolated antigen-binding protein or said chimeric antigen receptor.

In certain embodiments, the polypeptide molecule comprises a fusion protein.

In another aspect, the present application further provides an immunoconjugate comprising the isolated antigen-binding protein.

In another aspect, the present application further provides one or more isolated nucleic acid molecules encoding said isolated antigen-binding protein, said chimeric antigen receptor or said polypeptide molecule.

In certain embodiments, said nucleic acid molecule comprises a nucleotide sequence as set forth in any one of SEQ ID NO: 26, SEQ ID NO: 27, SEQ ID NO: 28, SEQ ID NO: 29, SEQ ID NO: 30, SEQ ID NO: 31, SEQ ID NO: 32, and SEQ ID NO: 33.

In certain embodiments, said nucleic acid molecule comprises any set of nucleotide sequences selected from the groups consisting of:
1) a nucleotide sequence as set forth in SEQ ID NO: 26 and a nucleotide sequence as set forth in SEQ ID NO: 27;
2) a nucleotide sequence as set forth in SEQ ID NO: 30 and a nucleotide sequence as set forth in SEQ ID NO: 27;
3) a nucleotide sequence as set forth in SEQ ID NO: 28 and a nucleotide sequence as set forth in SEQ ID NO: 29;
4) a nucleotide sequence as set forth in SEQ ID NO: 31 and a nucleotide sequence as set forth in SEQ ID NO: 32; and
5) a nucleotide sequence as set forth in SEQ ID NO: 31 and a nucleotide sequence as set forth in SEQ ID NO: 33.

In another aspect, the present application further provides a vector comprising said nucleic acid molecule.

In another aspect, the present application further provides a cell comprising said isolated antigen-binding protein, said chimeric antigen receptor, said polypeptide molecule, said nucleic acid molecule, or said vector.

In certain embodiments, said cell is an immune effector cell.

In certain embodiments, said cell includes a T cell, a B cell, a natural killer cell (NK cell), a macrophage, an NKT cell, a monocyte, a dendritic cell, a granulocyte, a lymphocyte, a leukocyte, a peripheral blood mononuclear cell, an embryonic stem cell, a lymphoid progenitor cell, and/or a pluripotent stem cell.

In certain embodiments, said cell is a T cell.

In certain embodiments, said cell further comprises and/or expresses a low-density lipoprotein receptor-related protein or a fragment thereof.

In certain embodiments, said low-density lipoprotein receptor-related protein or the fragment thereof comprises one or more groups selected from: low-density lipoprotein receptor-related proteins 1-12 and functional fragments thereof.

In certain embodiments, said low-density lipoprotein receptor-related protein or the fragment thereof is low-density lipoprotein receptor-related protein 5 and/or 6 or a fragment thereof.

In certain embodiments, said low-density lipoprotein receptor-related protein or the fragment thereof comprises an amino acid sequence as set forth in SEQ ID NO: 91.

In another aspect, the present application further provides a pharmaceutical composition, comprising said isolated antigen-binding protein, said chimeric antigen receptor, said polypeptide molecule, said immunoconjugate, said nucleic acid molecule, said vector, and/or or the cell, and optionally a pharmaceutically acceptable carrier.

In another aspect, the present application further provides a method for preparing the isolated antigen-binding protein, wherein said method comprises culturing said cell, under the condition that said antigen-binding protein is expressed.

In another aspect, the present application further provides a use of the isolated antigen-binding protein, the chimeric antigen receptor, the polypeptide molecule, the immunoconjugate, the nucleic acid molecule, the vector, the cell, and/or the pharmaceutical composition in the manufacture of a medicament, wherein said medicament is used for the prevention, alleviation and/or treatment of a disease and/or a disorder.

In certain embodiments, said disease and/or the disorder includes cancers.

In certain embodiments, said cancers include solid tumors and/or hematological tumors.

In certain embodiments, said cancers include gastric cancer and/or colon cancer.

In another aspect, the present application further provides a method for detecting CLDN18.2 in a sample, said method comprising: administering the isolated antigen-binding protein, the chimeric antigen receptor, the polypeptide molecule, the immunoconjugate, the nucleic acid molecule, the vector, the cell, and/or the pharmaceutical composition.

In another aspect, the present application further provides a reagent or a kit for detecting CLDN18.2 in a sample, comprising the isolated antigen-binding protein, the chimeric antigen receptor, the polypeptide molecule, the immunoconjugate, the nucleic acid molecule, the vector, the cell, and/or the pharmaceutical composition.

In another aspect, the present application further provides a use of the isolated antigen-binding protein, the chimeric antigen receptor, the polypeptide molecule, the immunoconjugate, the nucleic acid molecule, the vector, the cell, and/or the pharmaceutical composition in preparation of a kit, and the kit is used for detecting the presence and/or content of CLDN18.2 in a sample.

Those skilled in the art can easily perceive other aspects and advantages of the present application from the following detailed description. Only exemplary embodiments of the present application are shown and described in the following detailed description. As those skilled in the art will appreciate, from the content of the present application, those skilled in the art may make changes to the disclosed specific embodiments without departing from the spirit and scope of the invention to which the present application relates. Accordingly, the descriptions in the drawings and specification of this application are only exemplary and not restrictive.

### BRIEF DESCRIPTION OF THE DRAWINGS

The specific features of the invention referred to this application are shown in the appended claim. The features and advantages of the invention to which the present application relates can be better understood with reference to the exemplary embodiments and the accompanying drawings described below in detail. The drawings are described briefly as follows.
FIG. 1 shows the binding activity of the antigen-binding proteins of the present application to cells with high expression of human CLDN18.2, tested by FACS;
FIG. 2 shows the binding activity of the antigen-binding proteins of the present application to cells with high expression of CLDN18.1, tested by FACS;
FIG. 3 shows the binding activity of the antigen-binding proteins of the present application to a tumor cell line, tested by FACS;
FIG. 4 shows the species cross-binding activity analysis of the antigen-binding proteins of this application;
FIGS. 5a-5b show the CDC activity testing of the antigen-binding proteins of this application;
FIG. 6 shows the competitive binding activity analysis of the antigen-binding proteins of this application;
FIG. 7 shows the change in body weight of mice after administration;
FIG. 8 shows the relative change (%) in body weight of mice after administration;
FIG. 9 shows the change in tumor volume of mice after administration;
FIG. 10 shows the tumor inhibition rate of mice after administration;
FIG. 11 shows the survival curves of mice after administration;
FIG. 12A-B shows identification of the antigen-binding activity of CLDN18.2 single-chain antibodies;
FIG. 13 shows identification of the binding activity of CLDN18.2 single-chain antibodies to non-target cells;
FIG. 14 shows the anti-tumor activity of the CLDN18.2 antigen-binding proteins of this application in a mouse model of human gastric cancer;
FIGS. 15A-D show the expression of CLDN18.2-specific CAR in T cell;
FIG. 16 shows the in vitro killing activity testing of CAR-T cell;
FIGS. 17A-B show cytokines secretion testing of CLDN18.2-specific CAR;
FIG. 18A-D shows the anti-tumor effect of CLDN18.2-specific CAR-T cell in a mouse colon cancer CDX model.

### DETAILED DESCRIPTION

Embodiments of the invention of the present application will be described in the following specific embodiments, and those skilled in the art can easily understand other advantages and effects of the invention of the present application from the content disclosed herein.

### Definition of Terms

In the present application, the terms "CLDN18.2" and "Claudin18.2" are used interchangeably and generally refer to cell junction protein Claudin18 isoform 2. The term encompasses "full-length", unprocessed CLDN18.2 as well as any form of CLDN18.2 resulting from cellular processing. CLDN18.2 may include complete CLDN18.2 and fragments thereof, functional variants, isoforms, species homologues, derivatives, analogs and analogs having at least one epitope in common with CLDN18.2. The amino acid sequence of CLDN18.2 (e.g., human CLDN18.2) may be known in the art. For example, the nucleotide sequence of human CLDN18.2 may be shown under GeneBank Accession No. NM_001002026.3. For example, the nucleotide sequence of mouse CLDN18.2 may be shown under GeneBank Accession No. NM_001194921.1. For example, the nucleotide sequence of cynomolgus monkey CLDN18.2 may be shown under GeneBank Accession No. XM_001114708.4.

In the present application, the terms "CLDN18.1" and "Claudin18.1" are used interchangeably and generally refer to cell junction protein Claudin18 isoform 1. The term encompasses "full-length", unprocessed CLDN18.1 as well as any form of CLDN18.2 resulting from cellular processing. CLDN18.2 may include complete CLDN18.1 and fragments thereof, functional variants, isoforms, species homologues, derivatives, analogs and analogs having at least one epitope in common with CLDN18.1. The amino acid sequence of CLDN18.1 (e.g., human CLDN18.1) may be known in the art. For example, the nucleotide sequence of human CLDN18.1 may be shown under GeneBank Accession No. NM_016369.4. For example, the nucleotide sequence of mouse CLDN18.1 may be shown under GeneBank Accession No. NM_019815.3. For example, the nucleotide sequence of cynomolgus monkey CLDN18.1 may be shown under GeneBank Accession No. XM_005545863.2.

In the present application, the term "isolated" generally refers to that material is obtained from its natural state by artificial means. If an "isolated" substance or component occurs in nature, it may be that its natural environment has been altered, the substance has been isolated from its natural environment, or both. For example, a naturally-occurring polynucleotide or polypeptide present in a living animal is not isolated, but the same high-purity polynucleotide or polypeptide, separated from this natural environment, is isolated. The term "isolated" does not exclude the admixture of artificial or synthetic substances, or the presence of other impure substances which do not affect the activity of the substance.

In the present application, the term "isolated antigen-binding protein" generally refers to a protein with antigen-binding ability obtained from its natural state by artificial means. The "isolated antigen-binding protein" may include an antigen-binding moiety and, optionally, a framework or framework portion that allows the antigen-binding moiety to adopt a conformation that facilitates binding of the antigen-binding moiety to an antigen. The antigen-binding protein may comprise, for example, antibody-derived protein framework regions (FRs) or alternative protein framework regions or artificial framework regions with grafted CDRs or CDR derivatives. Such frameworks include, but are not limited to, antibody-derived framework regions comprising mutations introduced, for example, to stabilize the three-dimensional structure of the antigen-binding protein, and fully synthetic framework regions comprising, for example, biocompatible polymers. (see, e.g., Korndorfer, et al., 2003, Proteins: Structure, Function, and Bioinformatics,53 (1): 121-129 (2003); Roque, et al., Biotechnol. Prog. 20: 639-654 (2004)). Examples of the antigen-binding protein include, but are not limited to: human antibodies, humanized antibodies; chimeric antibodies; recombinant antibodies; single-chain antibodies; bispecific antibody; triple functional antibody; four-functional bodies; Fab, Fab', Fv fragments, Fv(ab')₂, F(ab)₂, scFv, di-scFv, dAb, and IgD antibodies; IgE antibodies; IgM antibodies; IgGl antibodies; IgG2 antibodies; IgG3 antibodies; or IgG4 antibodies and fragments thereof.

In the present application, the terms "variable domain" and "variable region" are used interchangeably and generally refer to a portion of a heavy and/or light chain of an antibody. The variable domains of the heavy and light chains may be referred to as "V_{H}" and "V_{L}," respectively (or "VH" and "VL," respectively). These domains are typically the most variable parts of an antibody (relative to other antibodies of the same class) and contain the antigen-binding site.

In the present application, the term "variable" typically refers to that some segments of the variable domain may be quite different in sequence between antibodies. The variable domains mediate antigen binding and determine the specificity of a particular antibody to its particular antigen. However, the variability is not evenly distributed throughout the variable domains. It is typically concentrated in three segments, known as hypervariable regions (CDRs or HVRs), in the light-chain and heavy-chain variable domains. A more highly conserved portion of the variable domain is called a framework region (FR). The variable domains of naturally occurring heavy and light chains each comprise four FR regions, most adopting a β-folding configuration, connected by three CDRs, which form a circular connection and in some cases form part of the β- folding structure. The CDRs in each chain are closely brought together by the FR, and form, together with the CDRs from another chain, an antigen-binding site of the antibody (see Kabat et al., Sequences of Immunological Interest, Fifth Edition, National Institute of Health, Bethesda, Md. (1991)).

In the present application, the term "antibody" generally refers to an immunoglobulin or a fragment or derivative thereof and encompasses any polypeptide comprising an antigen-binding site, whether produced in vitro or in vivo. The term includes, but is not limited to, polyclonal, monoclonal, monospecific, multispecific, non-specific, humanized, single-chain, chimeric, synthetic, recombinant, hybrid, mutated and transplanted antibodies. Unless otherwise modified by the term "intact", as in "intact antibody", for the purposes of the present invention, the term "antibody" also includes antibody fragments, such as Fab, F (ab') ₂, Fv, scFv, Fd, dAb and other antibody fragments that maintain an antigen binding function (e.g., specific binding to human CLDN18.2). Typically, such fragments will include the antigen binding domains. A basic 4-chain antibody unit is a heterotetrameric glycoprotein composed of two identical light (L) chains and two identical heavy (H) chains. An IgM antibody consists of five basic heterotetrameric units and another polypeptide called J chain, and contain 10 antigen-binding sites, while an IgA antibody includes two to five 4-chain antibody units that can bind to J chains to form a multivalent combination. For an IgG, the 4-chain unit is typically about 150,000 Daltons. Each L chain is linked to an H chain by a covalent disulfide bond, while two H chains are linked to each other by one or more disulfide bonds depending on the H chain isotype. Each H or L chain also has regularly spaced intrachain disulfide bridges. Each H chain has a variable domain (VH) at the N terminal, followed by three constant domains (CH) for α and γ chains respectively and four CH domains for µ and ε isoforms respectively. Each L chain has a variable domain (VL) at the N terminal and a constant domain at its other end. The VL corresponds to the VH, and the CL corresponds to the first constant domain (CH1) of the heavy chain. Certain amino acid residues are believed to form an interface between the light-chain and heavy-chain variable domains. A VH and a VL pair together to form a single antigen-binding site. For the structure and properties of the different classes of antibodies, see, e.g., Basic and Clinical Immunology, 8th Edition, Daniel P. Sties, Abba I. Terr and Tristram G Parsolw (eds), Appleton & Lange, Norwalk, Conn., 1994, page 71 and chapter 6. L chains from any vertebrate species can be classified into one of two distinct types, called κ and λ, based on the amino acid sequences of their constant domains. Immunoglobulins can be divided into different classes or isotypes based on the amino acid sequence of the heavy chain (CH) constant domain. There are currently five classes of immunoglobulins: IgA, IgD, IgE, lgG, and IgM, with heavy chains designated α, δ, ε, γ and µ, respectively. The γ and α classes are further divided into subclasses based on relatively small differences in CH sequence and function. For example, humans express the following subclasses: IgG1, IgG2A, IgG2B, IgG3, IgG4, IgA1, and IgK1.

In the present application, the term "CDR", also called "complementarity determining region", generally refers to a region in the variable domain of an antibody, the sequence of which is highly variable and/or forms a structure-defining loop. Generally, there are six CDRs in an antibody, three (HCDR1, HCDR2, and HCDR3) in a VH and three (LCDR1, LCDR2, and LCDR3) in a VL. In certain embodiments, naturally occurring camelid antibodies consisting only of heavy chains are capable of functioning and stabilizing in the absence of light chains (see, for example, Hamers-Casterman et al., Nature 363: 446-448 (1993); Sheriff et al., Nature Struct. Biol. 3:733-736 (1996)).

In the present application, the term "framework region" or "FR" generally refers to the more highly conserved portions of antibody variable domains, known as the framework regions. Typically, there are four FRs (H-FR1, H-FR2, H-FR3 and H-FR4) in each naturally occurring heavy-chain variable region, and four FRs (L-FR1, L-FR2, L-FR3 and L-FR4) in each naturally occurring light-chain variable region. For example, the VL of the isolated antigen-binding protein described in the present application may comprise an L-FR1, an L-FR2, an L-FR3, and an L-FR4. The VH of the isolated antigen-binding protein described in the present application may comprise an H-FR1, an H-FR2, an H-FR3, and an H-FR4.

In the present application, the term "antigen-binding fragment" generally refers to one or more fragments that have the ability to specifically bind to an antigen (e.g., CLDN18.2). In the present application, the antigen-binding fragment may include Fab, Fab', F(ab)₂, Fv fragments, F(ab')₂, scFv, di-scFv and/or dAb.

In the present application, the term "Fab" generally refers to an antigen-binding fragment of an antibody. As described above, intact antibodies can be digested using papain as described above. The antibody is digested with papain to produce two identical antigen-binding fragments, i.e., a "Fab" fragment and a residual "Fc" fragment (i.e., the Fc region, ibid.). The Fab fragment may consist of an intact L chain, the variable region of a heavy chain and the first constant region (C_{H}1) of the H chain (V_{H}).

In the present application, the term "Fab' fragment" generally refers to a monovalent antigen-binding fragment of a human monoclonal antibody, which is slightly larger than the Fab fragment. For example, the Fab' fragment may comprise all of the light chain, all of the variable domains of the heavy chain, and all or part of the first and second constant domains of the heavy chain. For example, the Fab' fragment may also comprise part or all of the 220-330 amino acid residues of a heavy chain.

In the present application, the term "F(ab')2" generally refers to antibody fragments produced by pepsin digestion of intact antibodies. The F(ab')2 fragment comprises two Fab fragments and part of the hinge region held together by disulfide bonds. The F(ab')2 fragments have bivalent antigen-binding activity and are capable of cross-linking antigens.

In the present application, the term "Fv fragment" used herein generally refers to a monovalent antigen-binding fragment of a human monoclonal antibody, comprising all or part of the heavy-chain and light-chain variable regions and lacking the heavy-chain and light-chain constant regions. The heavy-chain and light-chain variable regions include, for example, CDRs. For example, an Fv fragment comprises all or part of amino-terminal variable regions of about 110 amino acids of the heavy and light chains.

In the present application, the term "scFv" generally refers to a fusion protein comprising at least one antibody fragment comprising a variable region of a light chain and at least one antibody fragment comprising a variable region of a heavy chain, where the light-chain and heavy-chain variable regions are contiguous (e.g. via a synthetic linker such as a short flexible polypeptide linker) and can be expressed as a single-chain polypeptide. Moreover, the scFv retains the specificity of the intact antibody from which it is derived. Unless otherwise specified, in the present application, the scFv can have the VL and VH regions in any order (e.g., relative to the N terminal and C terminal of the polypeptide), and the scFv may comprise VL-linker-VH or VH-Linker-VL.

In the present application, the term "dAb" generally refers to an antigen-binding fragment having a VH domain and a VL domain, or having a VH domain or a VL domain (see, e.g., Ward et al., (Nature,1989 Oct 12; 341 (6242): 544-6); see Holt et al., Trends Biotechnol.,2003,21 (11): 484-490; and see, e.g., WO 06/030220, WO 06/003388 and other patent applications disclosed by Domantis Ltd.)

In the present application, the term "monoclonal antibody" generally refers to an antibody molecule preparation composed of a single molecule. The monoclonal antibody is generally highly specific for a single antigenic site. Moreover, unlike conventional polyclonal antibody preparations, which often have different antibodies directed against different determinants, each monoclonal antibody is directed against a single determinant on the antigen. In addition to their specificity, the advantage of monoclonal antibodies lies in that they may be synthesized by hybridoma culture, without being contaminated by other immunoglobulins. The modifier "monoclonal" indicates the characteristics of an antibody obtained from a substantially homogeneous population of antibodies and is not construed as requiring that the antibody be produced by any particular method. For example, the monoclonal antibodies used herein may be prepared in hybridoma cells, or may be prepared by recombinant DNA methods.

In the present application, the term "chimeric antibody" generally refers to an antibody in which the variable region is derived from one species and the constant region is derived from another species. Generally, the variable region is derived from an antibody ("parent antibody") in an experimental animal such as a rodent, and the constant region is derived from a human antibody, such that the resulting chimeric antibody is less likely to elicit an adverse immune response in an individual human than the parental (e.g., mouse-derived) antibody.

In the present application, the term "humanized antibody" generally refers to an antibody in which some or all of the amino acids outside the CDR of a non-human antibody (e.g., a mouse antibody) have been replaced by corresponding amino acids derived from human immunoglobulins. In the CDR, small additions, deletions, insertions, substitutions or modifications of amino acids may also be allowed so long as they still retain the ability of the antibody to bind to a specific antigen. A humanized antibody may optionally comprise at least a portion of a constant region of a human immunoglobulin. The "humanized antibody" retains antigen specificity similar to that of the original antibody. The "humanized" form of a non-human antibody (e.g., a murine antibody) may minimally comprise a chimeric antibody derived from a non-human immunoglobulin sequence. In some cases, CDR residues in a human immunoglobulin (receptor antibody) may be replaced with CDR residues from a nonhuman species (donor antibody) (such as a mouse, a rat, a rabbit, or a non-human primate) with the desired properties, affinity, and/or capability. In some cases, FR residues of the human immunoglobulin may be replaced with corresponding non-human residues. In addition, the humanized antibody may comprise amino acid modifications that are absent in the recipient antibody or in the donor antibody. These modifications may be made to further improve the properties, such as binding affinity, of the antibody.

In the present application, the term "fully human antibody" generally refers to an antibody that comprises only human immunoglobulin protein sequences. The fully human antibody may comprise murine sugar chains if it is produced in mice, in mouse cells, or in hybridomas derived from mouse cells. Similarly, a "mouse antibody" or "rat antibody" refers to an antibody comprising only mouse or rat immunoglobulin sequences, respectively. The fully human antibody can be produced in humans and in transgenic animals with human immunoglobulin germline sequences, by phage display technology or other molecular biology methods. Exemplary technologies that can be used to make antibodies have been described in US Patents 6,150,584, 6,458,592, and 6,420,140. Other techniques, such as using libraries, are known in the art.

In the present application, the term "directly linked" is contrasted with the term "indirectly linked" and generally refers to direct connection. For example, the "directly linked" may refer to a case where substances are directly linked without a spacer. The spacer may be a linker. For example, the linker may be a peptide linker. The term "indirectly linked" generally refers to a case where substances are not directly linked. For example, the linking may refer to a case of connection by a spacer. For example, in the isolated antigen-binding protein, the C terminal of the L-FR1 can be directly or indirectly linked to the terminal of the LCDR1.

In the present application, the term "isolated nucleic acid molecule" generally refers to nucleotides, deoxyribonucleotides or ribonucleotides of any length in an isolated form, or analogs isolated from their natural environment or artificially synthesized.

In the present application, the term "vector" generally refers to a nucleic acid delivery vehicle into which a polynucleotide encoding a protein can be inserted such that the protein can be expressed. The vector can be expressed by transforming, transducing or transfecting the host cell, so that the genetic material elements carried thereon can be expressed in the host cell. For example, vectors may include: plasmids; phagemids; cosmids; artificial chromosomes such as yeast artificial chromosome (YAC), bacterial artificial chromosome (BAC) or P1-derived artificial chromosome (PAC); phage such as λ phage or M13 phages and animal viruses, etc. Animal virus species used as vectors may include retroviruses (including lentiviruses), adenoviruses, adeno-associated viruses, herpesviruses (such as herpes simplex virus), poxviruses, baculoviruses, papillomaviruses, and papovavirus (e.g., SV40). A vector may comprise a variety of elements that control expression, including promoter sequence, transcriptional initiation sequence, enhancer sequence, selective element and reporter gene. In addition, the vector may also comprise replication initiation sites. The vector may also comprise components that assist its entry into cells, such as, but not exclusively, viral particles, liposomes, or protein coats.

In the present application, the term "cell" generally refers to an individual cell, a cell line or a cell culture that can be or has been the receptor of a subject's plasmid or vector, and it comprises the nucleic acid molecule of the present invention or the vector of the present invention. The cell may include a progeny of an individual cell. Due to natural, accidental or deliberate mutations, the progeny cell may not be necessarily identical (either in the morphology of the total DNA complement or in the genome) to the original parent cell. The cell may include cells transfected in vitro with vectors described herein. The cell may refer to bacterial cells (e.g., E. coli), yeast cells, or other eukaryotic cells, such as COS cells, Chinese Hamster Ovary (CHO) cells, CHO-K1 cells, LNCAP cells, HeLa cells, HEK293 cells, COS-1 cells, NS0 cells or stem cells (such as ES cells, iPS cells, and mesenchymal stem cells) or immune cells (such as T cells, NK cells, NKT cells, and macrophages). In certain embodiments, the cell refers to a mammalian cell. In certain embodiments, the mammalian cell is HEK293 cell.

In the present application, the term "pharmaceutical composition" generally refers to a composition for preventing/treating a disease or a disorder. The pharmaceutical composition may comprise the isolated antigen-binding protein of this application, the nucleic acid molecule of this application, the vector of this application and/or the cell of this application, and optionally a pharmaceutically acceptable adjuvant. In addition, the pharmaceutical composition may also comprise suitable formulations of one or more (pharmaceutically effective) carriers, stabilizers, excipients, diluents, solubilizers, surfactants, emulsifiers and/or preservatives. The acceptable components of the composition are preferably nontoxic to recipients at the dosages and concentrations employed. The pharmaceutical composition of this application includes, but is not limited to, liquid, frozen and lyophilized compositions.

In the present application, the term "pharmaceutically acceptable carrier" generally includes pharmaceutically acceptable carriers, excipients or stabilizers that are nontoxic to cells or mammals to which they are exposed at the dosages and concentrations employed. The physiologically acceptable carriers may include, for example, buffers; antioxidants; low-molecular-weight (less than about 10 residues) polypeptides; proteins; hydrophilic polymers; amino acids; monosaccharides; disaccharides and other carbohydrates; chelating agents; sugar alcohols; salt-forming counterions such as sodium; and/or nonionic surfactants.

In the present application, the term "specifically binding" or "specific" generally refers to a measurable and reproducible interaction, such as the binding between a target and an antibody, whereby the existence of a target may be determined in the presence of a heterogeneous population of molecules (including biomolecules). For example, an antibody specifically binding to a target (which may be an epitope) may be an antibody that binds to that target with greater affinity, avidity, easier, and/or for a longer duration than it binds to other targets. In certain embodiments, the antibody specifically binds to an epitope on a protein that is conservative among proteins of different species. In certain embodiments, the "specifically binding" includes, but does not require exclusive binding.

In the present application, the term "reference antibody" generally refers to an antibody that can bind to an antigen (e.g., CLDN18.2). In some cases, the antigen-binding protein used herein does not have competitive binding activity compared to the reference antibody. In some cases, the reference antibody used herein may be zolbetuximab.

In the present application, the term "substantially not binding" generally means not binding or binding with very weak binding activity. The very weak binding activity used herein may mean, for example, that according to the flow cytometry for detecting binding activity, the average fluorescence intensity of an antibody binding to CLDN18.1 is at least about 50%, about 55%, about 60%, about 70%, about 80%, about 90%, about 91%, about 91%, about 93%, about 94%, about 95%, about 96%, about 97%, about 98%, about 99%, or about 100% lower than that of a positive control for the CLDN18.1 antibody.

In the present application, the term "subject" generally refers to human or non-human animals, including but not limited to cats, dogs, horses, pigs, cows, sheep, rabbits, mice, rats or monkeys.

In the present application, the term "tumor" generally refers to a neoplasm or a solid lesion formed by abnormal cell growth. The tumor in the present application may be a solid tumor or a hematological tumor. For example, the tumor in the present may be a CLDN18.2-positive tumor.

The term "cancer" generally refers to a disease characterized by the rapid and uncontrolled growth of abnormal cells. Cancer cells may spread to other parts of the body locally or through the bloodstream and lymphatic system. The cancer in the present application includes, but is not limited to, gastric cancer and/or colon cancer, etc. The terms "tumor" and "cancer" are used interchangeably herein, for example, both terms encompass solid tumors and liquid tumors, for example, diffuse or circulating tumors. In the present application, the term "cancer" or "tumor" may include premalignant and malignant cancers and tumors.

In the present application, the proteins, polypeptides and/or amino acid sequences involved should also be understood to include at least the following scope: variants or homologues having the same or similar functions as the proteins or polypeptides.

In the present application, the variant may be, for example, a protein or polypeptide formed by substituting, deleting or adding one or more amino acids in the amino acid sequence of the defined protein and/or polypeptide (e.g., the antibody or a fragment thereof that specifically binds to the CLDN18.2 protein). For example, the functional variant may comprise a protein or polypeptide with amino acid changes induced by substituting, deleting, and/or inserting at least one amino acid, for example, 1-30, 1-20, or 1-10 amino acids, and for another example, 1, 2, 3, 4, or 5 amino acids. The functional variant may substantially maintain the biological properties of the protein or polypeptide before the changes (e.g., substitution, deletion, or addition). For example, the functional variants may maintain at least 60%, 70%, 80%, 90%, or 100% of the biological activity (e.g., the antigen-binding ability) of the protein or the polypeptide before the changes. For example, the substitution may be conservative substitution.

In the present application, the homologue used herein may be a protein or polypeptide that has at least about 85% (for example, at least about 85%, about 90%, about 91%, about 92%, about 93%, about 94%, about 95%, about 96%, about 97%, about 98%, about 99% or higher) sequence homology with the amino acid sequence of the defined protein and/or polypeptide (e.g., an antibody or a fragment thereof that specifically binds to the CLDN18.2 protein).

In the present application, the homology generally refers to the similarity, resemblance or association between two or more sequences. The "percentage of sequence homology" may be calculated in the following way: comparing the two sequences to be aligned in a comparison window; determining, in the two sequences, the number of positions at which identical nucleic acid bases (for example, A, T, C, G, I) or identical amino acid residues (for example, Ala, Pro, Ser, Thr, Gly, Val, Leu, Ile, Phe, Tyr, Trp, Lys, Arg, His, Asp, Glu, Asn, Gln, Cys, and Met) exist to acquire the number of matching positions; and dividing the number of matching positions by the total number of positions in the comparison window (i.e., the window size); and multiplying a result by 100 to produce the percentage of sequence homology. The alignment for determining the percentage of sequence homology may be achieved in a variety of ways known in the art, for example, by using publicly available computer software such as BLAST, BLAST-2, ALIGN or Megalign (DNASTAR) software. Those skilled in the art may determine appropriate parameters for sequence alignment, including any algorithm required to implement the maximum alignment undergoing comparison, within a full-length sequence range or within a target sequence region. The homology may also be determined by the following methods: FASTA and BLAST. For the description of the FASTA algorithm, a reference may be made to W. R. Pearson and D. J. Lipman, "Improved Tools for Biological Sequence Comparison", Proc. Natl. Acad. Sci., 85: 2444-2448, 1988: and D. J. Lipman and W. R. Pearson, "Rapid and Sensitive Protein Similarity Searches", Science, 227: 1435-1441, 1989. For the description of the BLAST algorithm, a reference may be made to S. Altschul, W. Gish, W. Miller, E. W. Myers and D. Lipman, "Basic Local Alignment Search Tool", Journal of Molecular Biology, 215: 403-410, 1990.

In this application, the term "comprise" generally refers to the meaning of including, inclusive, containing, or encompassing. In some cases, it also means "is/are" and "consist of'.

In the present application, the term "about" generally refers to a variation within a range of 0.5%-10% above or below a specified value, for example, a variation within a range of 0.5%, 1%, 1.5%, 2%, 2.5%, 3%, 3.5%, 4%, 4.5%, 5%, 5.5%, 6%, 6.5%, 7%, 7.5%, 8%, 8.5%, 9%, 9.5% or 10% above or below a specified value.

### DETAILED DESCRIPTION

The CDR of an antibody, also known as the complementarity determining region, is a portion of the variable region. The amino acid residues in this region may make contacts with the antigen or antigenic epitope. The CDR of an antibody can be determined by a variety of coding systems, such as CCG, Kabat, Chothia, and IMGT, and Kabat/Chothia may be preferred after comprehensive consideration. These coding systems are known in the art (see, e.g., http://www.bioinf.org.uk/abs/index.html#kabatnum). Those skilled in the art can use different coding systems to determine the CDR according to the sequence and structure of the antibody. There may be differences in the CDRs using different coding systems. In this application, the CDR covers CDR sequences classified according to any CDR classification method. It also covers its variants, which are obtained by substitution, deletion and/or addition of one or more amino acids to the amino acid sequence of the CDR. The variants may include substitutions, deletions and/or additions of, for example, 1-30, 1-20 or 1-10 amino acids, and for example, 1, 2, 3, 4, 5, 6, 7, 8 or 9 amino acids, to the amino acid sequences of the CDR. The CDR may also include its homologues having amino acid sequences at least about 85% (e.g., at least about 85%, about 90%, about 91%, about 92%, about 93%, about 94%, about 95%, about 96%, about 97%, about 98%, about 99% or higher) identical to the amino acid sequence of the CDR.

In one aspect, the present application provides an isolated antigen-binding protein. The isolated antigen-binding protein may comprise an HCDR3. In the present application, said HCDR3 may comprise an amino acid sequence as set forth in SEQ ID NO: 67. For example, said HCDR3 may comprise an amino acid sequence as set forth in SEQ ID NO: 4. For example, said HCDR3 may comprise an amino acid sequence as set forth in SEQ ID NO: 12. For example, said HCDR3 may comprise an amino acid sequence as set forth in SEQ ID NO: 18.

In the present application, the isolated antigen-binding protein may comprise an HCDR2. In the present application, said HCDR2 may comprise an amino acid sequence as set forth in SEQ ID NO: 68. In the present application, said HCDR2 may comprise an amino acid sequence as set forth in SEQ ID NO: 11. For example, said HCDR2 may comprise an amino acid sequence as set forth in SEQ ID NO: 3. For example, said HCDR2 may comprise an amino acid sequence as set forth in SEQ ID NO: 17.

In the present application, the isolated antigen-binding protein may comprise an HCDR1. In the present application, said HCDR1 may comprise an amino acid sequence as set forth in SEQ ID NO: 2. In the present application, said HCDR1 may comprise an amino acid sequence as set forth in SEQ ID NO: 10. In the present application, said HCDR1 may comprise an amino acid sequence as set forth in SEQ ID NO: 16.

In the present application, the isolated antigen-binding protein may comprise an HCDR1, an HCDR2 and an HCDR3. In the present application, said HCDR1 may comprise an amino acid sequence as set forth in any one of SEQ ID NO: 2, SEQ ID NO: 10, and SEQ ID NO: 16; said HCDR2 may comprise an amino acid sequence as set forth in SEQ ID NO: 68 or SEQ ID NO: 11; and said HCDR3 may comprise an amino acid sequence as set forth in SEQ ID NO: 67.

In the present application, the HCDR1 of said isolated antigen-binding protein may comprise an amino acid sequence as set forth in any one of SEQ ID NO: 2, SEQ ID NO: 10, and SEQ ID NO: 16; said HCDR2 may comprise an amino acid sequence as set forth in any one of SEQ ID NO: 3, SEQ ID NO: 17, and SEQ ID NO: 11; and said HCDR3 may comprise an amino acid sequence as set forth in any one of SEQ ID NO: 4, SEQ ID NO: 12, and SEQ ID NO: 18.

In the present application, the isolated antigen-binding protein may comprise any set of HCDR1, HCDR2 and HCDR3, selected from the groups consisting of:
1) said HCDR1 comprising an amino acid sequence as set forth in SEQ ID NO: 2, said HCDR2 comprising an amino acid sequence as set forth in SEQ ID NO: 3, and said HCDR3 comprising an amino acid sequence as set forth in SEQ ID NO: 4;
2) said HCDR1 comprising an amino acid sequence as set forth in SEQ ID NO: 10, said HCDR2 comprising an amino acid sequence as set forth in SEQ ID NO: 11, and said HCDR3 comprising an amino acid sequence as set forth in SEQ ID NO: 12; and
3) said HCDR1 comprising an amino acid sequence as set forth in SEQ ID NO: 16, said HCDR2 comprising an amino acid sequence as set forth in SEQ ID NO: 17, and said HCDR3 comprising an amino acid sequence as set forth in SEQ ID NO: 18.

In the present application, the isolated antigen-binding protein may comprise an H-FR1, wherein a C terminal of said H-FR1 is directly or indirectly linked to an N terminal of said HCDR1, and said H-FR1 may comprise an amino acid sequence as set forth in SEQ ID NO: 72. For example, said H-FR1 may comprise an amino acid sequence as set forth in SEQ ID NO: 34. For example, said H-FR1 may comprise an amino acid sequence as set forth in SEQ ID NO: 43. For example, said H-FR1 may comprise an amino acid sequence as set forth in SEQ ID NO: 49.

In the present application, the isolated antigen-binding protein may comprise an H-FR2, and said H-FR2 is located between said HCDR1 and said HCDR2 and comprises an amino acid sequence as set forth in SEQ ID NO: 73. For example, said H-FR2 may comprise an amino acid sequence as set forth in SEQ ID NO: 35. For example, said H-FR2 may comprise an amino acid sequence as set forth in SEQ ID NO: 44.

In the present application, the isolated antigen-binding protein may comprise an H-FR3,, and said H-FR3 is located between said HCDR2 and said HCDR3 and comprises an amino acid sequence as set forth in SEQ ID NO: 80. In the present application, said H-FR3 may comprise an amino acid sequence as set forth in SEQ ID NO: 45. For example, said H-FR3 may comprise an amino acid sequence as set forth in SEQ ID NO: 36. For example, said H-FR3 may comprise an amino acid sequence as set forth in SEQ ID NO: 42.

In the present application, the isolated antigen-binding protein may comprise an H-FR4, wherein an N terminal of said H-FR4 is linked to a C terminal of said HCDR3, and said H-FR4 comprises an amino acid sequence as set forth in SEQ ID NO: 74. For example, said H-FR4 may comprise an amino acid sequence as set forth in SEQ ID NO: 46. For example, said H-FR4 may comprise an amino acid sequence as set forth in SEQ ID NO: 37.

In the present application, the isolated antigen-binding protein may comprise an H-FR1, an H-FR2, an H-FR3, and an H-FR4. In the present application, said H-FR1 may comprise an amino acid sequence as set forth in SEQ ID NO: 72; said H-FR2 may comprise an amino acid sequence as set forth in SEQ ID NO: 73; said H-FR3 may comprise an amino acid sequence as set forth in SEQ ID NO: 80 or SEQ ID NO: 45; and said H-FR4 may comprise an amino acid sequence as set forth in SEQ ID NO: 74.

In the present application, said H-FR1 may comprise an amino acid sequence as set forth in any one of SEQ ID NO: 34, SEQ ID NO: 43 and SEQ ID NO: 49; said H-FR2 may comprise an amino acid sequence as set forth in SEQ ID NO: 35 or SEQ ID NO: 44; said H-FR3 may comprise an amino acid sequence as set forth in SEQ ID NO: 36 or SEQ ID NO: 42; and said H-FR4 may comprise an amino acid sequence as set forth in SEQ ID NO: 46 or SEQ ID NO: 37.

In the present application, the isolated antigen-binding protein may comprise any set of H-FR1, H-FR2, H-FR3, and H-FR4, selected from the groups consisting of:
1) said H-FR1 comprising an amino acid sequence as set forth in SEQ ID NO: 34, said H-FR2 comprising an amino acid sequence as set forth in SEQ ID NO: 35; said H-FR3 comprising an amino acid sequence as set forth in SEQ ID NO: 36; and said H-FR4 comprising an amino acid sequence as set forth in SEQ ID NO: 37;
2) said H-FR1 comprising an amino acid sequence as set forth in SEQ ID NO: 43, said H-FR2 comprising an amino acid sequence as set forth in SEQ ID NO: 44; said H-FR3 comprising an amino acid sequence as set forth in SEQ ID NO: 45; and said H-FR4 comprising an amino acid sequence as set forth in SEQ ID NO: 46;
3) said H-FR1 comprising an amino acid sequence as set forth in SEQ ID NO: 34, said H-FR2 comprising an amino acid sequence as set forth in SEQ ID NO: 35; said H-FR3 comprising an amino acid sequence as set forth in SEQ ID NO: 42; and said H-FR4 comprising an amino acid sequence as set forth in SEQ ID NO: 37; and
4) said H-FR1 comprising an amino acid sequence as set forth in SEQ ID NO: 49, said H-FR2 comprising an amino acid sequence as set forth in SEQ ID NO: 35; said H-FR3 comprising an amino acid sequence as set forth in SEQ ID NO: 42; and said H-FR4 comprising an amino acid sequence as set forth in SEQ ID NO: 37.

In the present application, the isolated antigen-binding protein may comprise an HCDR1, an HCDR2, an HCDR3, an H-FR1, an H-FR2, an H-FR3, and an H-FR4. For example, said HCDR1, HCDR2, HCDR3, H-FR1, H-FR2, H-FR3, and H-FR4 may respectively and sequentially comprise amino acid sequences as set forth in: SEQ ID NO: 2, SEQ ID NO: 3, SEQ ID NO: 4, SEQ ID NO: 34, SEQ ID NO: 35, SEQ ID NO: 36, and SEQ ID NO: 37; SEQ ID NO: 10, SEQ ID NO: 11, SEQ ID NO: 12, SEQ ID NO: 43, SEQ ID NO: 44, SEQ ID NO: 45, and SEQ ID NO: 46; SEQ ID NO: 16, SEQ ID NO: 17, SEQ ID NO: 18, SEQ ID NO: 34, SEQ ID NO: 35, SEQ ID NO: 42, and SEQ ID NO: 37; or SEQ ID NO: 16, SEQ ID NO: 17, SEQ ID NO: 18, SEQ ID NO: 49, SEQ ID NO: 35, SEQ ID NO: 42, and SEQ ID NO: 37.

In the present application, the isolated antigen-binding protein may comprise a VH. In the present application, said VH may comprise an amino acid sequence as set forth in SEQ ID NO: 78. For example, said VH may comprise an amino acid sequence as set forth in SEQ ID NO: 1. For example, said VH may comprise an amino acid sequence as set forth in SEQ ID NO: 9. For example, said VH may comprise an amino acid sequence as set forth in SEQ ID NO: 15. For example, said VH may comprise an amino acid sequence as set forth in SEQ ID NO: 19.

In the present application, the isolated antigen-binding protein may comprise an LCDR3. In the present application, said LCDR3 may comprise an amino acid sequence as set forth in SEQ ID NO: 69. For example, said LCDR3 may comprise an amino acid sequence as set forth in SEQ ID NO: 8. For example, said LCDR3 may comprise an amino acid sequence as set forth in SEQ ID NO: 14. For example, said LCDR3 may comprise an amino acid sequence as set forth in SEQ ID NO: 23. For example, said LCDR3 may comprise an amino acid sequence as set forth in SEQ ID NO: 25.

In the present application, the isolated antigen-binding protein may comprise an LCDR2. In the present application, said LCDR2 may comprise an amino acid sequence as set forth in SEQ ID NO: 70. For example, said LCDR2 may comprise an amino acid sequence as set forth in SEQ ID NO: 7. For example, said LCDR2 may comprise an amino acid sequence as set forth in SEQ ID NO: 22.

In the present application, the isolated antigen-binding protein may comprise an LCDR1. In the present application, said LCDR1 may comprise an amino acid sequence as set forth in SEQ ID NO: 71. For example, said LCDR1 may comprise an amino acid sequence as set forth in SEQ ID NO: 6. For example, said LCDR1 may comprise an amino acid sequence as set forth in SEQ ID NO: 21.

In the present application, the isolated antigen-binding protein may also comprise an LCDR1, an LCDR2 and an LCDR3. For example, said LCDR1 may comprise an amino acid sequence as set forth in SEQ ID NO: 71; said LCDR2 may comprise the amino acid sequence as set forth in SEQ ID NO: 70; and said LCDR3 can comprise the amino acid sequence as set forth in SEQ ID NO: 69.

In the present application, in the isolated antigen-binding protein, said LCDR1 may comprise an amino acid sequence as set forth in SEQ ID NO: 6 or SEQ ID NO: 21; said LCDR2 may comprise an amino acid sequence as set forth in SEQ ID NO: 7 or SEQ ID NO: 22; and said LCDR3 may comprise an amino acid sequence as set forth in any one of SEQ ID NO: 8, SEQ ID NO: 14, SEQ ID NO: 23, and SEQ ID NO: 25.

In the present application, the isolated antigen-binding protein may comprise any set of LCDR1, LCDR2 and LCDR3, selected from the groups consisting of:
1) said LCDR1 comprising an amino acid sequence as set forth in SEQ ID NO: 6, said LCDR2 comprising an amino acid sequence as set forth in SEQ ID NO: 7, and said LCDR3 comprising an amino acid sequence as set forth in SEQ ID NO: 8;
2) said LCDR1 comprising an amino acid sequence as set forth in SEQ ID NO: 6, said LCDR2 comprising an amino acid sequence as set forth in SEQ ID NO: 7, and said LCDR3 comprising an amino acid sequence as set forth in SEQ ID NO: 14;
3) said LCDR1 comprising an amino acid sequence as set forth in SEQ ID NO: 21, said LCDR2 comprising an amino acid sequence as set forth in SEQ ID NO: 22; and said LCDR3 comprising an amino acid sequence as set forth in SEQ ID NO: 23; and
4) said LCDR1 comprising an amino acid sequence as set forth in SEQ ID NO: 6, said LCDR2 comprising an amino acid sequence as set forth in SEQ ID NO: 7, and said LCDR3 comprising an amino acid sequence as set forth in SEQ ID NO: 25.

In the present application, the isolated antigen-binding protein may comprise an L-FR1, and a C terminal of said L-FR1 is directly or indirectly linked to an N terminal of said LCDR1. In the present application, said L-FR1 may comprise an amino acid sequence as set forth in SEQ ID NO: 75. For example, said L-FR1 may comprise an amino acid sequence as set forth in SEQ ID NO: 38. For example, said L-FR1 may comprise an amino acid sequence as set forth in SEQ ID NO: 47. For example, said L-FR1 may comprise an amino acid sequence as set forth in SEQ ID NO: 50. For example, said L-FR1 may comprise an amino acid sequence as set forth in SEQ ID NO: 53.

In the present application, the isolated antigen-binding protein may comprise an L-FR2, and said L-FR2 is located between the LCDR1 and the LCDR2. In the present application, said L-FR2 may comprise an amino acid sequence as set forth in SEQ ID NO: 39.

In the present application, the isolated antigen-binding protein can comprise an L-FR3, and said L-FR3 is located between the LCDR2 and the LCDR3. In the present application, said L-FR3 may comprise an amino acid sequence as set forth in SEQ ID NO: 76. For example, said L-FR3 may comprise an amino acid sequence as set forth in SEQ ID NO: 40. For example, said L-FR3 may comprise an amino acid sequence as set forth in SEQ ID NO: 48. For example, said L-FR3 can comprise an amino acid sequence as set forth in SEQ ID NO: 51.

In the present application, the isolated antigen-binding protein may comprise an L-FR4, and an N terminal of said L-FR4 is linked to a C terminal of said LCDR3. In the present application, said L-FR4 may comprise an amino acid sequence as set forth in SEQ ID NO: 77. For example, said L-FR4 may comprise an amino acid sequence as set forth in SEQ ID NO: 41. For example, said L-FR4 may comprise an amino acid sequence as set forth in SEQ ID NO: 52. For example, said L-FR4 may comprise an amino acid sequence as set forth in SEQ ID NO: 54.

In the present application, the isolated antigen-binding protein may comprise an L-FR1, an L-FR2, an L-FR3, and an L-FR4. For example, said L-FR1 may comprise an amino acid sequence as set forth in SEQ ID NO: 75; said L-FR2 may comprise an amino acid sequence as set forth in SEQ ID NO: 39; said L-FR3 may comprise an amino acid sequence as set forth in SEQ ID NO: 76; and said L-FR4 may comprise an amino acid sequence as set forth in SEQ ID NO: 77.

In the present application, said L-FR1 of the isolated antigen-binding protein may comprise an amino acid sequence as set forth in any one of SEQ ID NO: 38, SEQ ID NO: 47, SEQ ID NO: 50, and SEQ ID NO: 53; said L-FR2 may comprise an amino acid sequence as set forth in SEQ ID NO: 39; said L-FR3 may comprise an amino acid sequence as set forth in any one of SEQ ID NO: 40, SEQ ID NO: 48, and SEQ ID NO: 51; and said L-FR4 may comprise an amino acid sequence as set forth in any one of SEQ ID NO: 41, SEQ ID NO: 52, and SEQ ID NO: 54.

In the present application, the isolated antigen-binding protein may comprise any set of L-FR1, L-FR2, L-FR3, and L-FR4, selected from the groups consisting of:
1) said L-FR1 comprising an amino acid sequence as set forth in SEQ ID NO: 38, said L-FR2 comprising an amino acid sequence as set forth in SEQ ID NO: 39, said L-FR3 comprising an amino acid sequence as set forth in SEQ ID NO: 40, and said L-FR4 comprising an amino acid sequence as set forth in SEQ ID NO: 41;
2) said L-FR1 comprising an amino acid sequence as set forth in SEQ ID NO: 47, said L-FR2 comprising an amino acid sequence as set forth in SEQ ID NO: 39; said L-FR3 comprising an amino acid sequence as set forth in SEQ ID NO: 48, and said L-FR4 comprising an amino acid sequence as set forth in SEQ ID NO: 41;
3) said L-FR1 comprising an amino acid sequence as set forth in SEQ ID NO: 50, said L-FR2 may comprise an amino acid sequence as set forth in SEQ ID NO: 39; said L-FR3 comprising an amino acid sequence as set forth in SEQ ID NO: 51, and said L-FR4 comprising an amino acid sequence as set forth in SEQ ID NO: 52; and
4) said L-FR1 comprising an amino acid sequence as set forth in SEQ ID NO: 53, said L-FR2 comprising an amino acid sequence as set forth in SEQ ID NO: 39; said L-FR3 comprising an amino acid sequence as set forth in SEQ ID NO: 40, and said L-FR4 comprising an amino acid sequence as set forth in SEQ ID NO: 54.

In the present application, the isolated antigen-binding protein may comprise an LCDR1, an LCDR2, an LCDR3, an L-FR1, an L-FR2, an L-FR3, and an L-FR4. For example, said LCDR1, LCDR2, LCDR3, L-FR1, L-FR2, L-FR3, and L-FR4 may respectively and sequentially comprise amino acid sequences as set forth in: SEQ ID NO: 6, SEQ ID NO: 7, SEQ ID NO: 8, SEQ ID NO: 38, SEQ ID NO: 39, SEQ ID NO: 40, and SEQ ID NO: 41; SEQ ID NO: 6, SEQ ID NO: 7, SEQ ID NO: 14, SEQ ID NO: 47, SEQ ID NO: 39, SEQ ID NO: 48, and SEQ ID NO: 41; SEQ ID NO: 21, SEQ ID NO: 22, SEQ ID NO: 23, SEQ ID NO: 50, SEQ ID NO: 39, SEQ ID NO: 51, and SEQ ID NO: 52; or SEQ ID NO: 6, SEQ ID NO: 7, SEQ ID NO: 25, SEQ ID NO: 53, SEQ ID NO: 39, SEQ ID NO: 40, and SEQ ID NO: 54.

In the present application, the isolated antigen-binding protein may comprise a VL. In the present application, said VL may comprise an amino acid sequence as set forth in SEQ ID NO: 79. For example, said VL may comprise an amino acid sequence as set forth in SEQ ID NO: 5. For example, said VL may comprise an amino acid sequence as set forth in SEQ ID NO: 13. For example, said VL may comprise an amino acid sequence as set forth in SEQ ID NO: 20. For example, said VL may comprise an amino acid sequence as set forth in SEQ ID NO: 24.

In the present application, the isolated antigen-binding protein may comprise an HCDR1, an HCDR2, an HCDR3, an LCDR1, an LCDR2 and an LCDR3. In the present application, said HCDR1 may comprise an amino acid sequence as set forth in any one of SEQ ID NO: 2, SEQ ID NO: 10, and SEQ ID NO: 16; said HCDR2 may comprise an amino acid sequence as set forth in SEQ ID NO: 68 or SEQ ID NO: 11; said HCDR3 may comprise an amino acid sequence as set forth in SEQ ID NO: 67; said LCDR1 may comprise an amino acid sequence as set forth in SEQ ID NO: 71; said LCDR2 may comprise the amino acid sequence as set forth in SEQ ID NO: 70; and said LCDR3 may comprise the amino acid sequence as set forth in SEQ ID NO: 69.

In the present application, said HCDR1 may comprise an amino acid sequence as set forth in any one of SEQ ID NO: 2, SEQ ID NO: 10, and SEQ ID NO: 16; said HCDR2 may comprise an amino acid sequence as set forth in any one of SEQ ID NO: 3, SEQ ID NO: 17, and SEQ ID NO: 11; said HCDR3 may comprise an amino acid sequence as set forth in any one of SEQ ID NO: 4, SEQ ID NO: 12, and SEQ ID NO: 18; said LCDR1 may comprise an amino acid sequence as set forth in SEQ ID NO: 6 or SEQ ID NO: 21; said LCDR2 comprising an amino acid sequence as set forth in SEQ ID NO: 7 or SEQ ID NO: 22; and said LCDR3 comprising an amino acid sequence as set forth in any one of SEQ ID NO: 8, SEQ ID NO: 14, SEQ ID NO: 23, and SEQ ID NO: 25.

In the present application, the isolated antigen-binding protein may comprise any set of HCDR1, HCDR2, HCDR3, LCDR1, LCDR2 and LCDR3, selected from the groups consisting of:
1) said HCDR1 comprising an amino acid sequence as set forth in SEQ ID NO: 2, said HCDR2 comprising an amino acid sequence as set forth in SEQ ID NO: 3, said HCDR3 comprising an amino acid sequence as set forth in SEQ ID NO: 4, said LCDR1 comprising an amino acid sequence as set forth in SEQ ID NO: 6, said LCDR2 comprising the amino acid sequence as set forth in SEQ ID NO: 7, and said LCDR3 comprising an amino acid sequence as set forth in SEQ ID NO: 8;
2) said HCDR1 comprising an amino acid sequence as set forth in SEQ ID NO: 10, said HCDR2 comprising an amino acid sequence as set forth in SEQ ID NO: 11, said HCDR3 comprising an amino acid sequence as set forth in SEQ ID NO: 12, said LCDR1 comprising an amino acid sequence as set forth in SEQ ID NO: 6, said LCDR2 comprising the amino acid sequence as set forth in SEQ ID NO: 7, and said LCDR3 comprising an amino acid sequence as set forth in SEQ ID NO: 14;
3) said HCDR1 comprising an amino acid sequence as set forth in SEQ ID NO: 16, said HCDR2 comprising an amino acid sequence as set forth in SEQ ID NO: 17, said HCDR3 comprising an amino acid sequence as set forth in SEQ ID NO: 18, said LCDR1 comprising an amino acid sequence as set forth in SEQ ID NO: 6, said LCDR2 comprising an amino acid sequence as set forth in SEQ ID NO: 7, and said LCDR3 comprising an amino acid sequence as set forth in SEQ ID NO: 8;
4) said HCDR1 comprising an amino acid sequence as set forth in SEQ ID NO: 16, said HCDR2 comprising an amino acid sequence as set forth in SEQ ID NO: 17, said HCDR3 comprising an amino acid sequence as set forth in SEQ ID NO: 18, said LCDR1 comprising an amino acid sequence as set forth in SEQ ID NO: 21, said LCDR2 comprising an amino acid sequence as set forth in SEQ ID NO: 22; and said LCDR3 comprising an amino acid sequence as set forth in SEQ ID NO: 23; and
5) said HCDR1 comprising an amino acid sequence as set forth in SEQ ID NO: 16, said HCDR2 comprising an amino acid sequence as set forth in SEQ ID NO: 17, said HCDR3 comprising an amino acid sequence as set forth in SEQ ID NO: 18, said LCDR1 comprising an amino acid sequence as set forth in SEQ ID NO: 6, said LCDR2 comprising an amino acid sequence as set forth in SEQ ID NO: 7, and said LCDR3 comprising an amino acid sequence as set forth in SEQ ID NO: 25.

In the present application, the isolated antigen-binding protein may comprise a VH and a VL. In the present application, said VH may comprise an amino acid sequence as set forth in SEQ ID NO: 78, and said VL may comprise an amino acid sequence as set forth in SEQ ID NO: 79.

In the present application, in the isolated antigen-binding protein, said VH may comprise an amino acid sequence as set forth in any one of SEQ ID NO: 1, SEQ ID NO: 9, SEQ ID NO: 15, and SEQ ID NO: 19, and said VL may comprise an amino acid sequence as set forth in any one of SEQ ID NO: 5, SEQ ID NO: 13, SEQ ID NO: 20, and SEQ ID NO: 24.

In the present application, the isolated antigen-binding protein may comprise any set of amino acid sequences selected from the groups consisting of:
1) said VH comprising an amino acid sequence as set forth in SEQ ID NO: 1 and said VL comprising an amino acid sequence as set forth in SEQ ID NO: 5;
2) said VH comprising an amino acid sequence as set forth in SEQ ID NO: 15 and said VL comprising an amino acid sequence as set forth in SEQ ID NO: 5;
3) said VH comprising an amino acid sequence as set forth in SEQ ID NO: 9 and said VL comprising an amino acid sequence as set forth in SEQ ID NO: 13;
4) said VH comprising an amino acid sequence as set forth in SEQ ID NO: 19 and said VL comprising an amino acid sequence as set forth in SEQ ID NO: 20; and
5) said VH comprising an amino acid sequence as set forth in SEQ ID NO: 19 and said VL comprising an amino acid sequence as set forth in SEQ ID NO: 24.

In the present application, the isolated antigen-binding protein may comprise an antibody heavy chain constant region. In the present application, said antibody heavy chain constant region may be derived from a human IgG heavy chain constant region. In certain embodiments, the isolated antigen-binding protein may comprise an antibody heavy chain constant region and said antibody heavy chain constant region may be derived from a human IgG1 heavy chain constant region.

In the present application, the isolated antigen-binding protein can comprise an antibody light chain constant region. Said antibody light chain constant region may be derived from a human Igκ constant region.

In addition, it should be noted that the isolated antigen-binding protein of the present application may comprise heavy and/or light chain sequences with one or more conservative sequence modifications. The so-called "conservative sequence modification" refers to the amino acid modification that will not significantly affect or change the binding properties of the antibody. Such conservative modifications include amino acid substitutions, additions and deletions. Modifications can be introduced into the isolated antigen-binding protein of the present application by standard techniques known in the art, such as point mutations and PCR-mediated mutagenesis. Conservative amino acid substitutions are substitutions of amino acid residues with amino acid residues with similar side chains. Groups of amino acid residues having similar side chains are known in the art. These groups of amino acid residues include amino acids with basic side chains (e.g., lysine, arginine, and histidine), acidic side chains (e.g., aspartic acid and glutamic acid), uncharged polar side chains (e.g., glycine, asparagine, glutamine, serine, threonine, tyrosine, cysteine, and tryptophan), nonpolar side chains (e.g., alanine, valine, leucine acid, isoleucine, proline, phenylalanine, and methionine), β-branched side chains (e.g. threonine, valine, and isoleucine) and aromatic side chains (e.g., tyrosine, phenylalanine, tryptophan, and histidine). In certain embodiments, one or more amino acid residues in the CDR regions of the isolated antigen-binding protein of the present application may be replaced with other amino acid residues from the same side chain group. Those skilled in the art know that some conservative sequence modifications do not abolish antigen binding, see, e.g., Brummelletal., et al., (1993) Biochem 32:1180-8; de Wildt et al., (1997) Prot. Eng. 10: 835-41; Komissarov et al., (1997) J. Biol. Chem. 272: 26864-26870; Hall et al., (1992) J. Immunol. 149: 1605-12; Kelley and O'Connell (1993) Biochem.32: 6862-35; Adib-Conquy et al., (1998) Int. Immunol. 10: 341-6 and Beers et al., (2000) Clin. Can. Res. 6: 2835-43.

The physical/chemical properties and/or biological activities of the CLDN18.2 antigen-binding proteins described herein can be identified, screened, or characterized by various assays known in the art.

In certain embodiments, for example, the antigen-binding activity of the antigen-binding protein or fusion protein of the present application can be tested by known methods such as enzyme-linked immunosorbent assay (ELISA), immunoblotting (e.g., Western blot), flow cytometry (e.g., FACS), immunohistochemistry, immunofluorescence, etc.

In the present application, the isolated antigen-binding protein is capable of specifically binding to a CLDN18.2 antigen. An antigen-binding protein "specifically binding" to a CLDN18.2 antigen will generally bind to CLDN18.2 but not bind to or substantially not bind to other proteins lacking the CLDN18.2 sequence. Whether an antigen-binding protein (e.g., an antibody) binds to a CLDN18.2 antigen can be determined using any assay known in the art. For example, FACS (Fluorescence Activated Cell Sorting) can be used to determine the specific binding activity of the isolated antigen-binding protein to CLDN18.2. In certain embodiments, the specific binding can be concentration-dependent binding. For example, in binding activity experiments by FACS, the average fluorescence intensity of CLDN18.2 increased with increase of CLDN18.2 antibody concentration.

In the present application, the antigen-binding protein can bind to a human CLDN18.2 protein. In certain instances, the antigen-binding proteins described herein may also cross-react with murine (e.g., mouse) and/or monkey (e.g., cynomolgus monkey) CLDN18.2. It can be tested by, e.g., FACS. In the present application, the term "cross-reactivity" generally refers to the ability of an antibody to react with homologous proteins from other species.

In the present application, the antigen-binding protein is capable of eliciting a CDC effect. For example, the CLDN18.2 antigen-binding protein of the present application can elicit a strong CDC effect against SP2/0-human CLDN18.2 cells. For example, the CLDN18.2 antigen-binding protein of the present application can elicit a strong CDC effect on MC38-human CLDN18.2 cells. For example, said "elicit" may be implemented in a dose-dependent manner.

In the present application, the isolated antigen-binding protein does not substantially compete with a reference antibody for binding to CLDN18.2, as verified in FACS. Said reference antibody may comprise a heavy chain variable region (VH) and a light chain variable region (VL). For example, said VH of the reference antibody may comprise an amino acid sequence as set forth in SEQ ID NO: 55, and its VL may comprise an amino acid sequence as set forth in SEQ ID NO: 56.

In the present application, the antigen-binding protein is capable of inhibiting tumor growth and/or proliferation of tumor cells. Said tumor may be a CLDN18.2-expressing tumor, for example, colon cancer. The cell of said colon cancer may be MC38-human CLDN18.2 cell.

### Chimeric antigen receptor

In another aspect, the present application further provides a chimeric antigen receptor (CAR). The chimeric antigen receptor (CAR) may comprise a targeting moiety binding to the CLDN18.2 protein. For example, said targeting moiety binding to the CLDN18.2 protein may be the antigen-binding protein of the present application. For example, said targeting moiety may be present in the form of a scFv. For example, said scFv may comprise the CDR, VH, and/or VL of the isolated antigen-binding protein. For example, the VH and the VL of said scFv may be linked by an amino acid sequence as set forth in SEQ ID NO: 89. For example, said scFv may comprise an amino acid sequence as set forth in SEQ ID NO: 90.

In the present application, in addition to the extracellular targeting moiety binding to the CLDN18.2 protein, said CAR may also comprise other domains.

In the present application, said CAR may comprise a costimulatory signaling domain, which may provide a stimulatory signal. For example, said costimulatory signaling domain may comprise an intracellular costimulatory signaling domain of one or more proteins selected from the group consisting of CD28, 4-1BB, CD27, CD2, CD7, CD8, OX40, CD226, DR3, SLAM, CDS, ICAM-1, NKG2D, NKG2C, B7-H3, 2B4, FcεRIγ, BTLA, GITR, HVEM, DAP10, DAP12, CD30, CD40, CD40L, TIM1, PD-1, LFA-1, LIGHT, JAML, CD244, CD100, ICOS, a ligand of CD83, CD40, and MyD88.

For example, said costimulatory signaling domain may be an intracellular costimulatory signaling domain derived from 4-1BB. For example, said costimulatory signaling domain may comprise an amino acid sequence as set forth in SEQ ID NO: 85.

In some cases, said CAR may comprise an intracellular signaling domain, which may comprise a domain containing at least one ITAM motif. Said intracellular signaling domain can transmit an activation signal into the interior of the cell. For example, said intracellular signaling domain may comprise an intracellular signaling domain derived from one or more proteins selected from the group consisting of CD3ζ, CD3δ, CD3γ, CD3ε, CD79a, CD79b, FcεRIγ, FcεRIβ, FcyRIIa, bovine leukemia virus gp30, Epstein-Barr virus (EBV) LMP2A, simian immunodeficiency virus PBj 14Nef, Kaposi's Sarcoma Associated-Herpesvirus (HSKV), DAP10, DAP-12, and other domains containing at least one ITAM.

For example, said intracellular signaling domain may be a signaling domain derived from CD3ζ. For example, said intracellular signaling domain may comprise an amino acid sequence as set forth in SEQ ID NO: 86.

In some cases, said CAR may comprise a transmembrane domain, which is a sequence in a cell surface protein that spans the cell membrane. Said transmembrane domain may comprise a hydrophobic alpha helix. Said transmembrane domain may be derived from any type I transmembrane protein. Said transmembrane domain may be a synthetic sequence predicted to form a hydrophobic helix. For example, said transmembrane domain comprises a transmembrane domain derived from one or more proteins selected from the group consisting of CD8, CD28, 4-1BB, CD4, CD27, CD7, PD-1, TRAC, TRBC, CD3ε, CD3ζ, CTLA-4, LAG-3, CD5, ICOS, OX40, NKG2D, 2B4, CD244, FcεRIγ, BTLA, CD30, GITR, HVEM, DAP10, CD2, NKG2C, LIGHT, DAP12, CD40L, TIM1, CD226, DR3, CD45, CD80, CD86, CD9, CD16, CD22, CD33, CD37, CD64, CD134, CD137, CD154, and SLAM.

For example, said transmembrane domain may be a transmembrane domain derived from CD8. For example, said transmembrane domain may comprise an amino acid sequence as set forth in SEQ ID NO: 84.

In some cases, said CAR may comprise a hinge region, which may be located between said extracellular targeting moiety and said transmembrane domain. For example, said hinge region may comprise the hinge region of one or more proteins selected from the group consisting of: CD28, IgG1, IgG4, IgD, 4-1BB, CD4, CD27, CD7, CD8, PD-1, ICOS, OX40, NKG2D, NKG2C, FcεRIγ, BTLA, GITR, DAP10, CD40L, TIM1, CD226, SLAM, CD30, and LIGHT.

For example, said hinge region may be a hinge region derived from CD8. For example, said hinge region may comprise an amino acid sequence as set forth in SEQ ID NO: 83.

In the present application, said CAR may further comprise a signal peptide at the N terminal of said targeting moiety binding to the CLDN18.2 protein. For example, said signal peptide may be a signal peptide derived from the CD8 protein. For example, the signal peptide may comprise an amino acid sequence as set forth in SEQ ID NO: 88.

In the present application, said CAR may further comprise a low-density lipoprotein receptor-related protein or a fragment thereof. For example, said low-density lipoprotein receptor-related protein or the fragment thereof may be located at the C terminal of said CAR. For example, said low-density lipoprotein receptor-related protein or the fragment thereof may comprise low-density lipoprotein receptor-related proteins 1-12 and functional fragments thereof. For example, said low-density lipoprotein receptor-related protein or the fragment thereof is low-density lipoprotein receptor-related protein 5 and/or 6 or a fragment thereof. For example, said low-density lipoprotein receptor-related protein or the fragment thereof may comprise an amino acid sequence as set forth in SEQ ID NO: 91.

In the present application, the sequence of said low-density lipoprotein receptor-related protein or the fragment thereof in the CAR may be linked to the C-terminal sequence of the CAR through a self-cleaving peptide (e.g., 2A peptides such as T2A, P2A, and E2A). For example, said low-density lipoprotein receptor-related protein or the fragment thereof may be linked to the C terminal of the intracellular signaling domain through T2A. For example, said cleaving peptide may comprise an amino acid sequence as set forth in SEQ ID NO: 87.

In the present application, from the N terminal to the C terminal, said CAR may sequentially comprise a targeting moiety (e.g., the antigen-binding protein) binding to the CLDN18.2 protein, said hinge region, said transmembrane domain, said costimulatory signaling domain, and said intracellular signaling domain. For example, from the N terminal to the C terminal, said CAR may sequentially comprise said scFv, said hinge region derived from CD8, the transmembrane domain derived from CD8, the costimulatory signaling domain derived from 4-1BB, and the intracellular signaling domain derived from CD3ζ.

In the present application, a vector capable of expressing said CAR or immune effector cell is also included. Said vector may sequentially comprise a nucleic acid molecule encoding the targeting moiety binding to the CLDN18.2 protein (e.g., the antigen-binding protein), a nucleic acid molecule encoding said hinge region, a nucleic acid molecule encoding said transmembrane domain, a nucleic acid molecule encoding the costimulatory signaling domain, a nucleic acid molecule encoding said intracellular signaling domain, and a nucleic acid molecule encoding said low-density lipoprotein receptor-related protein or the fragment thereof.

In the present application, a vector capable of expressing said CAR or immune effector cell is also included. Said vector may sequentially comprise a nucleic acid molecule encoding the targeting moiety binding to the CLDN18.2 protein (e.g., the antigen-binding protein), a nucleic acid molecule encoding said hinge region, a nucleic acid molecule encoding said transmembrane domain, a nucleic acid molecule encoding said costimulatory signaling domain, a nucleic acid molecule encoding said intracellular signaling domain, a nucleic acid molecule encoding said cleaving peptide, and a nucleic acid molecule encoding said low-density lipoprotein receptor-related protein or the fragment thereof.

### Polypeptide molecules, immunoconjugates, nucleic acid molecules, vectors, cells, and pharmaceutical compositions

In another aspect, the present application provides a polypeptide molecule which may comprise the isolated antigen-binding protein of the present application or the chimeric antigen receptor of the present application.

In the present application, said polypeptide molecule may comprise a fusion protein. For example, the isolated antigen-binding protein of the present application can be fused with other functional molecules (such as antibodies or receptor ligands) to form bispecific or multispecific molecules. Said bispecific or multispecific molecule can specifically bind to at least two different binding sites or targeting molecules. Said bispecific or multispecific molecules can be prepared by genetic modification, somatic cell hybridization or chemical methods. See, e.g., Kufer et al, cited supra; Cao and Suresh, Bioconjugate Chemistry, 9 (6), 635-644 (1998); and van Spriel et al, Immunology Today, 21 (8), 391-397 (2000).

In another aspect, the present application further provides an immunoconjugate which may comprise the isolated antigen-binding protein of the present application.

In the present application, the isolated antigen-binding protein of the present application or a fragment thereof can be linked to another agent, such as a chemotherapeutic agent, a toxin, an immunotherapeutic agent, an imaging probe, and a spectroscopic probe. The "linked" can be achieved by one or more covalent bonds, or non-covalent interactions, and can include chelation. A variety of linkers, which may be known in the art, can be used to form immunoconjugates. Additionally, the immunoconjugates can be provided in the form of fusion proteins, which can be expressed from polynucleotides encoding the immunoconjugates. Said immunoconjugates can also comprise, for example, antibody-drug conjugates (ADCs). In the ADCs, antibodies and therapeutic agents can be cross-linked by linkers that are cleavable, such as peptide, disulfide, or hydrazone linkers.

In another aspect, the present application provides one or more nucleic acid molecules that may encode an isolated antigen-binding protein of the present application or a chimeric antigen receptor of the present application. For example, the isolated nucleic acid molecule may be produced or synthesized by: (i) in vitro amplification, for example, by polymerase chain reaction (PCR) amplification; (ii) clonal recombination; (iii) purification, for example, by enzymatic digestion and gel electrophoresis fractionation; or (iv) synthesis, for example, by chemical synthesis.

In the present application, the isolated nucleic acid molecule may comprise an amino acid sequence as set forth in any one of SEQ ID NO: 26, SEQ ID NO: 27, SEQ ID NO: 28, SEQ ID NO: 29, SEQ ID NO: 30, SEQ ID NO: 31, SEQ ID NO: 32, and SEQ ID NO: 33.

In the present application, said nucleic acid molecule may comprise any set of nucleotide sequences selected from the groups consisting of:
1) a nucleotide sequence as set forth in SEQ ID NO: 26 and a nucleotide sequence as set forth in SEQ ID NO: 27;
2) a nucleotide sequence as set forth in SEQ ID NO: 30 and a nucleotide sequence as set forth in SEQ ID NO: 27;
3) a nucleotide sequence as set forth in SEQ ID NO: 28 and a nucleotide sequence as set forth in SEQ ID NO: 29;
4) a nucleotide sequence as set forth in SEQ ID NO: 31 and a nucleotide sequence as set forth in SEQ ID NO: 32; and
5) a nucleotide sequence as set forth in SEQ ID NO: 31 and a nucleotide sequence as set forth in SEQ ID NO: 33.

In another aspect, the present application provides a vector, which may comprise the nucleic acid molecule of the present application. In addition, said vector may further comprise other genes, for example, a marker gene that is allowed to select this vector in a suitable host cell and under a suitable condition. In addition, the vector may also comprise an expression control element that allows a coding region to be expressed correctly in a suitable host. Such a control element is well known to those skilled in the art, which, for example, may include a promoter, a ribosome binding site, an enhancer, and other control elements that regulate gene transcription or mRNA translation. Said vector can be expressed by transforming, transducing or transfecting the host cell, so that the genetic material elements carried thereon can be expressed in the host cell. Said vector may comprise, for example, a plasmid, a cosmid, a virus, a bacteriophage, or other vectors commonly used in, for example, genetic engineering. For example, said vector is an expression vector. In addition, said vector may also include components that assist its entry into cells, such as, but not exclusively, viral particles, liposomes, or protein coats.

In another aspect, the present application provides a cell, which may comprise the isolated antigen-binding protein of the present application, the chimeric antigen receptor of the present application, the polypeptide molecule of the present application, the nucleic acid molecule of the present application or the vector of the present application. In certain embodiments, each type of host cell or each host cell may comprise one or one type of the nucleic acid molecule or vector of the present application. In certain embodiments, each type of host cell or each host cell may comprise a plurality of (for example, 2 or more) or a plurality of types of (for example, 2 or more types of) vectors of the present application. For example, the vector of the present application may be introduced into the host cell, for example, a eukaryotic cell, such as a plant-originated cell, a fungal cell, or a yeast cell, etc. In certain embodiments, said cell may be a bacterial cell (e.g., E. coli), a yeast cell, or other eukaryotic cells. The vector of the present application can be introduced into the host cell by methods known in the art.

In certain embodiments, said cell may be an immune effector cell. In certain embodiments, said cell may include a T cell, a B cell, a natural killer cell (NK cell), a macrophage, an NKT cell, a monocyte, a dendritic cell, a granulocyte, a lymphocyte, a leukocyte, a peripheral blood mononuclear cell, an embryonic stem cell, a lymphoid progenitor cell, and/or a pluripotent stem cell.

In certain embodiments, said cell may be a T cell.

In the present application, said cell may comprise and/or express said CAR. In the present application, said cell may comprise and/or express said CAR and said low-density lipoprotein receptor-related protein or the fragment thereof.

In another aspect, the present application further provides a pharmaceutical composition which may comprise the isolated antigen-binding protein of the present application, the chimeric antigen receptor of the present application, the polypeptide molecule of the present application, the immunoconjugate of the present application, the nucleic acid molecule of the present application, the vector of the present application, and/or the cell of the present application, and optionally a pharmaceutically acceptable carrier.

In certain embodiments, the pharmaceutical composition may further comprise suitable formulations of one or more (pharmaceutically effective) adjuvants, stabilizers, excipients, diluents, solubilizers, surfactants, emulsifiers and/or preservatives. The acceptable components of the compositions are preferably nontoxic to recipients at the dosages and concentrations employed. The pharmaceutical composition of the present application includes, but is not limited to, liquid, frozen and lyophilized compositions.

In the present application, said pharmaceutical composition may further comprise more than one active compound, which generally refers to those active compounds with complementary activities that do not adversely affect each other. The type and effective amount of such medicine may depend, for example, on the amount and type of antagonists present in the drug preparation, as well as the clinical parameters of a subject.

In the present application, said pharmaceutically acceptable carrier may include any and all solvents, dispersion media, coatings, isotonic agents and absorption delaying agents compatible with drug administration, which are generally safe and non-toxic.

In certain embodiments, said pharmaceutical composition may be administered by routes including parenteral, transdermal, intracavity, intraarterial, intrathecal and/or intranasal administration or may be directly injected into tissues. For example, the pharmaceutical composition can be administered to a patient or subject by infusion or injection. In certain embodiments, said pharmaceutical composition may be administered by different means, such as intravenous, intraperitoneal, subcutaneous, intramuscular, topical or intradermal administration. In certain embodiments, said pharmaceutical composition can be administered without interruption. Said uninterrupted (or continuous) administration can be achieved by a small pump system worn by the patient to measure the influx of a therapeutic agent into a patient, as described in WO2015/036583.

### Preparation method

In another aspect, the present application provides a preparation method for the antigen-binding protein. Said method may comprise culturing the host cell of the present application under a condition that the antigen-binding protein is expressed. For example, an appropriate medium, an appropriate temperature, a culture time and the like may be used, and these methods are understood by those of ordinary skills in the art.

Any method suitable for producing a monoclonal antibody may be used to produce the antigen-binding protein of the present application. For example, animals may be immunized with linked or naturally occurring CLDN18.2 proteins or fragments thereof. Suitable immunization methods may be used, including adjuvants, immunostimulants, and repeated booster immunizations, and one or more routes may be used.

Any suitable form of CLDN18.2 can be used as an immunogen (antigen) to produce a non-human antibody specific for CLDN18.2 and to screen the biological activity of the antibody. The eliciting immunogen may be a full-length mature human CLDN18.2, comprising natural homodimers, or peptides containing single/multiple epitopes. The immunogen may be used alone, or in combination with one or more immunogenicity enhancers known in the art.

A chimeric human antibody may be selected from any class of immunoglobulins, including IgM, IgD, IgG, IgA, and IgE. In the present application, the antibody may be an IgG antibody, and an IgG1 subtype may be used. An essential constant domain sequence may be optimized by screening antibodies with the biological assays described in the Examples below, so as to produce the desired biological activity. Similarly, any type of light chain may be used in the compounds and methods herein. For example, a K chain or its variants may be used in the compounds and methods of the present application.

### Method and Use

In another aspect, the present application further provides a use of the isolated antigen-binding protein, the chimeric antigen receptor, the polypeptide molecule, the immunoconjugate, the nucleic acid molecule, the vector, the cell and/or the pharmaceutical composition of the present application in the manufacture of a medicament, wherein said medicament is used for the prevention, alleviation and/or treatment of a disease and/or a disorder.

In another aspect, the present application further provides a method for the prevention, alleviation and/or treatment of a disease and/or a disorder. Said method may comprise: administering the isolated antigen-binding protein, the chimeric antigen receptor, the polypeptide molecule, the immunoconjugate, the nucleic acid molecule, the vector, the cell and/or the pharmaceutical composition of the present application to a subject in need. In the present application, said administration may be carried out by different means, such as intravenous, intratumoral, intraperitoneal, subcutaneous, intramuscular, topical or intradermal administration.

In another aspect, the present application further provides a use of the isolated antigen-binding protein, the chimeric antigen receptor, the polypeptide molecule, the immunoconjugate, the nucleic acid molecule, the vector, the cell and/or the pharmaceutical composition of the present application for the prevention, alleviation and/or treatment of a disease and/or a disorder.

In the present application, said disease and/or the disorder may include cancers.

In the present application, said cancers may include solid tumors and/or hematological tumors.

In the present application, said cancers may include CLDN18.2-positive tumors.

In the present application, said cancers include gastric cancer and/or colon cancer.

In the present application, said isolated antigen-binding protein may be co-administered together with one or more other antibodies to effectively inhibit tumor growth in a subject. Said isolated antigen binding protein may also be co-administered with chemotherapeutic agents.

In another aspect, the present application further provides a method for detecting CLDN18.2 in a sample. Said method comprises administering the isolated antigen-binding protein of the present application, said chimeric antigen receptor, said polypeptide molecule, said immunoconjugate, said nucleic acid molecule, said vector, said cell and/or said pharmaceutical composition. Said method may be an ex vivo and/or in vitro method.

In another aspect, the present application further provides a reagent or kit for detecting CLDN18.2 in a sample, which may comprise said isolated antigen-binding protein, said chimeric antigen receptor, said polypeptide molecule, said immunoconjugate, said nucleic acid molecule, said vector, said cell and/or said pharmaceutical composition.

In another aspect, the present application further provides a use of said isolated antigen-binding protein, said chimeric antigen receptor, said polypeptide molecule, said immunoconjugate, said nucleic acid molecule, said vector, said cell and/or said pharmaceutical composition in preparation of the reagent or kit for detecting CLDN18.2.

In the present application, said reagent or kit can be used for detecting the presence and/or content of CLDN18.2 in a sample.

Without wishing to be bound by any particular theory, it is believed that the following examples are merely to illustrate the antigen-binding protein, preparation method, uses and the like of the present application, and are not intended to limit the scope of the present application.

### EXAMPLES

### Example 1 Preparation of positive control antibodies against CLDN18.1 and CLDN18.2

The used positive control antibody against CLDN18.1 was a commercially available anti-CLDN18 rabbit monoclonal antibody (abcam, Cat#ab203563). The antibody can identify both CLDN18.1 and CLDN18.2, and its antigen-binding site is located intracellularly. In the present application, this antibody was used as a positive control antibody against CLDN18.1 and named anti-Claudin18 antibody.

According to the full-length amino acid sequences (e.g., amino acid sequences as set forth in SEQ ID NO: 81 and SEQ ID NO: 82) of the heavy and light chains of the 175D10 clonal (IMAB362) antibody provided by the patent application CN103509114A, GENEWIZ Suzhou was entrusted to perform conventional gene synthesis and clone them into the eukaryotic expression vectors pCMV-k and pCMV-IgG1 (NDL) provided by our company. The constructed plasmid included: pCMV-IMAB362-VL and pCMV-IMAB362-VH. The above two vector plasmids were co-transfected into Expi293 cells at a ratio of 3:2 (VL:VH) for transient expression. The cell culture supernatant was harvested after 5 days and purified by Protein A affinity chromatography, and BCA protein assay was then performed to finally obtain the positive control antibody against CLDN18.2, named zolbetuximab.

### Example 2 Construction of stable cell lines with high expression of CLDN18.1 and CLDN18.2

According to the nucleotide sequences of human CLDN18.1, human CLDN18.2, mouse CLDN18.1, mouse CLDN18.2, cynomolgus monkey CLDN18.1, and cynomolgus monkey CLDN18.2 given in Table 1, GENEWIZ Suzhou was entrusted to perform conventional gene synthesis and clone them into the lentiviral expression vector pHAGE-full EF1a-IzsGreen provided by our company. The obtained plasmid-containing puncture bacteria were activated and then subjected to plasmid extraction. Our company's lentivirus packaging and infection system was then used to infect 293T, CHO, and SP2/0 cells with lentivirus respectively. After the infection efficiency of the cells obtained after infection was tested by FACS, the cells were monoclonalized by gradient dilution method and then screened and verified by FACS, and then stable monoclonal cell lines with high expression of CLDN18.1 and CLDN18.2 were obtained.

**Table 1 Nucleotide sequences of CLDN18.1 and CLDN18.2**

| Origin of CLDN18 | Genbank accession No. | Nucleotide sequence |
|---|---|---|
| Human CLDN18.1 | NM_016369.4 | SEQ ID NO: 61 |
| Human CLDN18.2 | NM_001002026.3 | SEQ ID NO: 62 |
| Mouse CLDN18.1 | NM_019815.3 | SEQ ID NO: 63 |
| Mouse CLDN18.2 | NM_001194921.1 | SEQ ID NO: 64 |
| Cynomolgus monkey CLDN18.1 | XM_005545863.2 | SEQ ID NO: 65 |
| Cynomolgus monkey CLDN18.2 | XM_001114708.4 | SEQ ID NO: 66 |

For FACS verification of the stable cell lines with high expression of CLDN18.2, 30,000 transfected cells were added in V-shaped 96-well plates, the positive antibody zolbetuximab was then added and PBS negative control wells were set up. After incubation at 4 °C for 1 hour, the 96-well plates were washed once with FACS buffer, and a secondary antibody (goat F(ab')2 anti-human IgG-Fc (DyLight^{®} 650) (abcam, Cat#ab98593) was then added. After incubation at 4 °C for 30 minutes, the 96-well plates were washed twice with FACS buffer, the fluorescence of cells was then tested by using iQue Screener flow cytometer (purchased from IntelliCyt Company), and monoclonal cells with the highest MFI value were picked for expansion culture and cryopreservation.

For FACS verification of the stable cell lines with high expression of CLDN18.1, the cells were treated by two methods. In one method, after counting, the cells were directly resuspended with the FACS buffer and loaded in the plates, and the primary antibody was the CLDN18.2 positive antibody zolbetuximab provided in Example 1. In the other method, after counting, the cells were subjected to fixation and membrane rupture treatment (4A Biotech, fixative solution FXP008 and membrane rupture agent FXP009), so that the antibody was able to bind to the antigen site located intracellularly, where the primary antibody used was the commercially available CLDN18 antibody provided in Example 1. In the meanwhile, the cells were tested on the flow cytometer, and monoclonal cells strongly binding to the commercially available CLDN18 antibody but not binding to zolbetuximab were picked for expansion culture and cryopreservation.

The constructed stable cell lines with high expression of CLDN18 were labeled as: 293T-human CLDN18.1, 293T-human CLDN18.2, CHO-human CLDN18.1, CHO-human CLDN18.2, SP2/0-human CLDN18.1, SP2/0-human CLDN18.2, 293T-mouse CLDN18.1, 293T-mouse CLDN18.2, 293T-cynomolgus monkey CLDN18.1, 293T-cynomolgus monkey CLDN18.2, which are all stable monoclonal cell lines.

### Example 3 Generation of murine CLDN18.2 antigen-binding proteins

### 1. Mouse immunization

Using the full-length nucleotide sequence of the first extracellular segment of human CLDN18.2, GENEWIZ Suzhou was entrusted to perform conventional gene synthesis and clone it into a eukaryotic expression vector provided by our company. The constructed plasmid was pCMV-CLDN18.2-D1. Plasmid extraction was then carried out on the obtained punctured bacterial liquid to obtain a DNA immunogen. The cellular immunogen was the SP2/0-human CLDN18.2 cell constructed in Example 2. In this experiment, a total of nine Balb/c mice were immunized. Six mice were immunized with a combination of cell antigens and DNA antigens, and the other three mice were immunized with whole cell immunization. The classic mouse immunization schedule was followed. After the mice were immunized twice, mouse serum was collected. The serum titers were determined by FACS with use of the 293T-human CLDN18.2 cell. Mice that met the titer requirements were selected for the final booster immunization. If the titers do not meet the requirements, the immunization is required to be carried out 1 to 2 times more until the serum titers meet the requirements. Three days after the final immunization, the mouse spleen tissue was collected under sterile conditions and ground into a spleen cell suspension. The spleen cells were subjected to red blood cell lysis, then aliquoted and frozen in a -80 °C refrigerator for later use.

### 2. Hybridoma cell fusion

Mouse myeloma cell SP2/0 was resuscitated in advance, cultured and passaged in DMEM containing 10% fetal calf serum at 37 °C and 5% CO₂. On the day of fusion, the PEG1450 solution (Sigma Aldrich, Cat#25322-68-3) and high-glucose DMEM were preheated in a 37 °C water bath in advance. The status of the SP2/0 cell was observed under a microscope. It is best for cells to be in the logarithmic growth phase. The cell solution was centrifuged at 400g for 5 minutes, and the SP2/0 cell was collected and then resuspended in an appropriate amount of the preheated DMEM and counted with trypan blue (the cell viability was required to be 90% or above), and then placed into a 37 °C water bath for later use. Mouse spleen cell was resuscitated in a 37 °C water bath and washed once with 20 mL of preheated DMEM. The cell solution was centrifuged at 400g for 5 minutes and the supernatant was discarded. 25 mL of preheated DMEM was added to resuspend the cell and after the cell suspension was diluted to a certain ratio, cell counting was carried out using a hemocytometer. The spleen cell and the SP2/0 cell were mixed according to the cell count ratio (spleen cell: SP2/0=4:1), and the cell solution was centrifuged at 400g for 5 minutes and the supernatant was discarded. 1 mL of preheated PEG1450 solution was added slowly to perform cell fusion. The cell solution was loaded in 96-well plates, each plate with a total amount of 1×10⁷ spleen cells. The culture medium was a high-glucose DMEM containing 10% FBS, 1% double antibody, and 2% HAT supplement and 10% ClonaCell^{™}-HY Medium C (STEMCELL, CAT#: 03803). The medium was changed the day before the test. The fresh medium after change was high-glucose DMEM containing 10% FBS, 1% double antibody, and 1% HT supplement. 24 hours later, the cell culture supernatant from the 96-well plates was taken for flow cytometry.

### 3. FACS of hybridoma cell

The 293T-human CLDN18.2 and CHO-human CLDN18.2 cells constructed in Example 2 were selected for the first round of primary FACS of the mother clone hybridoma cells obtained after fusion as follows: Cells were digested and counted separately, resuspended in a flow cytometry buffer (PBS containing 0.1% BSA) and adjusted to a cell density of 1×10⁶ cells/ml, and then added to V-bottom 96-well plates at 30 ul/well. The hybridoma cell culture supernatant was taken at 50 µl/well and added to V-bottom 96-well plates loaded with cell antigens, and in the meanwhile a positive control (5 ug/ml zolbetuximab antibody, 30 µl/well) and a negative control (PBS buffer, 30 µl/well) were set up. The cells were co-incubated at 4 °C for 1 hour and the plates were washed once with the flow cytometry buffer. A corresponding secondary antibody was added at 30 ul/well and co-incubated at 4 °C for 30 minutes and the plates were then washed with the flow cytometry buffer twice. After the cells were shaken loose, the FACS buffer was added at 25 µl/well, and FACS was then performed on the iQue Screener flow cytometer (purchased from IntelliCyt Company). The mother clones that were positive for CLDN18.2 in the primary sorting were picked for expansion culture in 24-well cell culture plates. Three days later, the cell supernatant was taken for re-sorting by FACS. Four types of cells, i.e., CHO-human CLDN18.2, CHO-human CLDN18.1, 293T-human CLDN18.2, and 293T-human CLDN18.1, were loaded into the plates separately to sort CLDN18.2-positive and CLDN18.1-negative hybridoma mother clones therefrom. The positive hybridoma mother clones were monocloned using a gradient dilution method. After two to three rounds of subcloning and FACS, a monoclonal hybridoma cell secreting a CLDN18.2-specific antibody was obtained. The monoclonal hybridoma cell produced positive binding to 293T-human CLDN18.2 and CHO-human CLDN18.2 cells and negative binding to 293T-human CLDN18.1 and CHO-human CLDN18.1 cells.

The results are shown in Table 2. The subclones that produced positive binding to CLDN18.2 in the hybridoma cell culture supernatant include: 3A6, 7E3, 5E6, 14E12 and 17B10, a total of five monoclonal cells.

**Table 2 FACS results of hybridoma clones**

| Hybridoma clone No. | Average fluorescence intensity | | | |
|---|---|---|---|---|
| | 293T-human CLDN18.2 | CHO-human CLDN18.2 | 293T-human CLDN18.1 (Permeable membrane treatment) | CHO-human CLDN18.1 (Permeable membrane treatment) |
| 5E6 | 107513.5 | 292506 | 4329 | 5370 |
| 3A6 | 100961.5 | 214920 | 4840.5 | 5100.5 |
| 7E3 | 48153 | 84783.5 | 3041.5 | 3382 |
| 14E12 | 16631 | 27582.5 | 2581 | 1859 |
| 17B10 | 30630.5 | 52129 | 2397 | 1682.5 |
| Positive control | 619086 | 1032236 | 438510 | 546174.5 |
| Negative control | 2390.75 | 2141.5 | 2612.75 | 3367.75 |

### Example 4 Variable region sequencing of murine CLDN18.2 antigen-binding proteins

Degenerate primer amplification method was used for sequencing to obtain the VH and VL sequences of murine CLDN18.2 antigen-binding protein. For the primers used, please refer to the PROTOCOL published by *Lotta von Boehmer (doi: 10.1038 nprot.2016.102).* GENEWIZ Suzhou was entrusted to conduct primer synthesis. Specifically, five CLDN18.2-positive monoclonal hybridoma cells in Example 3 were collected, the total RNA of the cells was extracted using QIAGEN RNeasy Plus Mini Kit, the integrity of the RNA was tested by 1% agarose gel electrophoresis, and the RNA concentration was determined by using a NanoDrop nucleic acid quantitative analyzer. 1 ug of RNA was reverse-transcribed into cDNA by using an RNA reverse transcription kit, and cDNA was stored at -20 °C for later use. Using 5ul of cDNA as a template, the PCR reaction system and the PCR procedures were set according to the operating instructions of PCR high-fidelity enzyme (TransGen Biotech Beijing, AP231), where the annealing temperature was set according to the gradient cooling method. 1ul of the first round PCR product was then directly used as the template to carry out the second round of PCR. The PCR reaction system and procedures were the same as above. All second-round PCR products were loaded for 1% agarose gel electrophoresis, the specific target band of appropriate size was cut out, and the gel was then recovered. A blunt-end cloning vector (TransGen Biotech Beijing, CB501) was linked, and transformed into competent cell Trans1-T1. Cells were loaded on 2YT plates, and the 2YT plates were placed upside down in a 37 °C incubator for incubation for 12-16 hours. Single colonies were picked with a pipette tip in a clean workbench. After the colonies were activated, GENEWIZ Suzhou was entrusted to carry out sequencing. After sequencing, the VH and VL gene sequences of the five hybridoma clones were obtained, as shown in Table 3.

**Table 3. VH and VL sequences of 5 hybridoma clones**

| Hybridoma clone | Amino acid sequence | | Nucleotide sequence | |
|---|---|---|---|---|
| | VL | VH | VL | VH |
| 5E6 | SEQ ID NO: 5 | SEQ ID NO: 1 | SEQ ID NO: 27 | SEQ ID NO: 26 |
| 3A6 | SEQ ID NO: 13 | SEQ ID NO: 9 | SEQ ID NO: 29 | SEQ ID NO: 28 |
| 7E3 | SEQ ID NO: 5 | SEQ ID NO: 15 | SEQ ID NO: 27 | SEQ ID NO: 30 |
| 14E12 | SEQ ID NO: 20 | SEQ ID NO: 19 | SEQ ID NO: 32 | SEQ ID NO: 31 |
| 17B10 | SEQ ID NO: 24 | SEQ ID NO: 19 | SEQ ID NO: 33 | SEQ ID NO: 31 |

### Example 5 Preparation of CLDN18.2 antigen-binding proteins

Using the five VH and VL sequences obtained by sequencing in Example 4 as templates, primers were designed respectively, and GENEWIZ Suzhou was entrusted to carry out primer synthesis. High-fidelity enzyme was used for PCR amplification, agarose gel electrophoresis and gel recovery. The recovered plasmid DNA was homologously recombined (Vazyme, C112) into the digested eukaryotic expression vector (pCMV-IgG1NDL and pCMV-κ) with human IgG1 constant region. GENEWIZ Suzhou was entrusted to perform sequencing to identify correct positive recombination vectors. After positive recombination vectors were identified as correct by the sequencing, the plasmid extraction was carried out, and the heavy and light chains were co-transfected into Expi293 cells. Five days later, centrifugation was carried out and the cell culture supernatant was collected and then purified by ProteinA affinity chromatography to obtain intact human and mouse IgG1 and Igκ antigen-binding proteins. These five antigen-binding proteins were named c5E6, c7E3, c3A6, c14E12, and c17B10, respectively.

### Example 6 Determination of specific binding activity of CLDN18.2 antigen-binding proteins to CLDN18.2

The specific binding activity of the above chimeric antibodies to target cells was tested by FACS on the iQue Screener (purchased from IntelliCyt Company) using PBS containing 0.1% BSA as a buffer, and chose three target cells: stably transformed cell line expressing human CLDN18.2, stably transformed cell line expressing human CLDN18.1, and a tumor cell line, were respectively selected for determining binding activity.

### 1. Testing of binding activity of the antigen-binding proteins prepared in Example 5 to high-expressing human CLDN18.2 cells by FACS

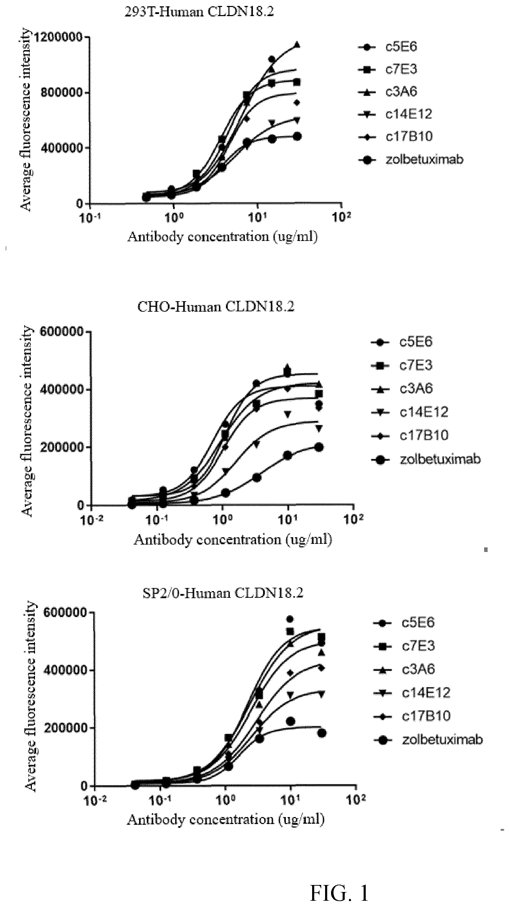

The cells used were 293T-human CLDN18.2, CHO-human CLDN18.2, and SP2/0-human CLDN18.2 cells constructed in Example 2. After digestion and counting, the cells were resuspended in a flow cytometry buffer, adjusted to the density of 1×10⁶ cells/ml, and then added into V-bottom 96-well plates at 30 µl/well. Primary antibodies were added at 30 µl/well. The antibodies with an initial concentration of 30 ug/ml were gradient diluted with the flow cytometry buffer at a two or three-fold ratio to form 7 gradients. A PBS negative control was set up for each antibody and the positive control antibody was zolbetuximab purified in Example 1. The cells were inoculated at 4 °C for one hour and then the plates were washed once with the flow cytometry buffer. The secondary antibody (abcam, Cat#ab98593) was added at 30 µl/well. After incubation at 4 °C for 30 minutes, the plates were washed twice with the flow cytometry buffer and the cells were shaken dispersedly. The flow cytometry buffer was then added at 25 µl/well, and then the cells were ready for FACS on the flow cytometer. Original data were entered into the software GraphPad8.0 for graphing and calculation. The results are shown in FIG. 1.

### 2. Testing of binding activity of the antigen-binding proteins prepared in Example 5 to high-expressing human CLDN18.1 cells by FACS

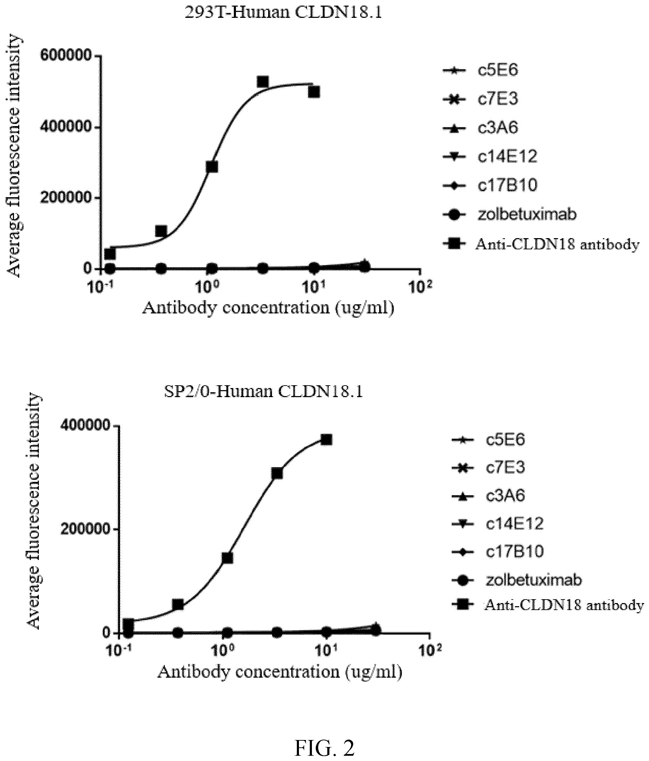

The positive control antibody used was a commercially available anti-Claudin18 antibody (abcam, Cat#ab203563). Since its antigen-binding site is located in the intracellular portion of the CLDN18.2 quaternary membrane protein, the FACS intracellular staining assay was required. Specifically, the cells used were 293T-human CLDN18.1 and SP2/0-human CLDN18.1 cells constructed in Example 1. After digestion and counting, the cells were fixed and membrane-broken. The treated cells were then resuspended in a flow cytometry buffer to the density of 1×10⁶ cells/ml, and then added into V-bottom 96-well plates at 30 µl/well. Primary antibodies were added at 30 µl/well. The antibodies with an initial concentration of 30 ug/ml were gradient diluted with the flow cytometry buffer at a three-fold ratio to form 7 gradients. A PBS negative control was set up for each antibody and the positive control antibody was diluted under the same condition as above. After inoculation at 4 °C for one hour, the plates were washed once with the flow cytometry buffer. The secondary antibodies (abcam, Cat#ab98593 and Cat#ab150079) were added at 30 µl/well. After incubation at 4 °C for 30 minutes, the plates were washed twice with the flow cytometry buffer and the cells were shaken loose. The flow cytometry buffer was added at 25ul/well, and then the cells were ready for FACS on the flow cytometer. Original data were entered into the software GraphPad8.0 for graphing and calculation. The results are shown in FIG. 2.

### 3. Testing of binding activity of the antigen-binding proteins prepared in Example 5 to a tumor cell line by FACS

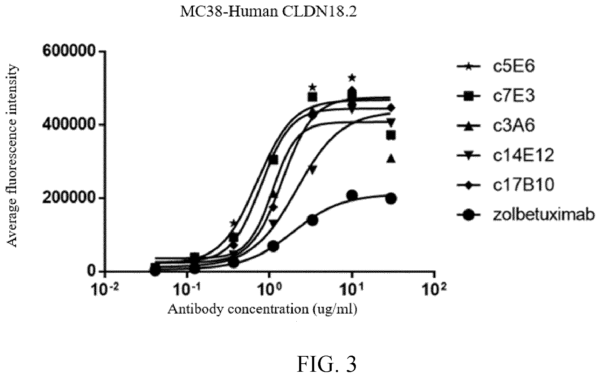

The stable MC38-human CLDN18.2 tumor cell with high expression of human CLDN18.2, which was constructed according to the method shown in Example 2, was selected as a target cell. After digestion and counting, cells were resuspended in a flow cytometry buffer, adjusted to the density of 1×10⁶ cells/ml, and then added into V-bottom 96-well plates at 30 µl/well. Primary antibodies were added at 30 µl/well. The antibodies with initial concentration of 30 ug/ml were gradient diluted with the flow cytometry buffer at a three-fold ratio to form 7 gradients. A PBS negative control was set up for each antibody and the positive control antibody was zolbetuximab purified in Example 1. The dilution was carried out under the same condition as above. After inoculation at 4 °C for one hour, the plates were washed once with the flow cytometry buffer. The secondary antibody (abcam, Cat#ab98593) was added at 30 µl/well. After incubation at 4 °C for 30 minutes, the plates were washed twice with the flow cytometry buffer and the cells were shaken dispersedly. The flow cytometry buffer was added at 25 µl/well, and then the cells were ready for FACS on the flow cytometer. Original data were entered into the software GraphPad8.0 for graphing and calculation. The results are shown in FIG. 3.

Through the above operation steps, five human and mouse antigen-binding proteins were expressed and purified, and their antigen-binding activities were verified by FACS. As shown in FIG. 1, the five antigen-binding proteins all show concentration-dependent binding activity to human CLDN18.2, and most of them show stronger binding activity than the positive antibody zolbetuximab. The test results of the three types of cells are consistent. As shown in FIG. 2, all the five antigen-binding proteins can specifically bind to human CLDN18.2 but not to human CLDN18.1. As shown in FIG. 3, all the five antigen-binding proteins strongly bind to the stable mouse colon cancer MC38 cell with high expression of human CLDN18.2, the binding activity intensity is concentration-dependent, and some of them have higher binding activity than the positive antibody zolbetuximab.

### Example 7 Analysis of species cross-binding activity of CLDN18.2 antigen-binding proteins

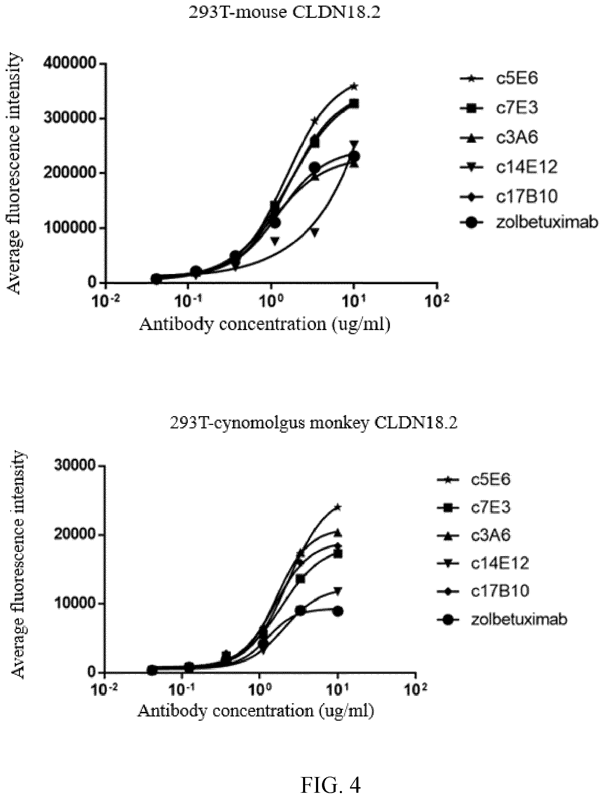

The species cross-binding activity of the above antigen-binding proteins to mouse CLDN18.2 and cynomolgus monkey CLDN18.2 were tested through FACS on the CytoFLEX flow cytometer (purchased from BECKMAN COULTER Company) using PBS containing 0.1% BSA as a buffer. Specifically, 293T-mouse CLDN18.2 and 293T-cynomolgus monkey CLDN18.2 cells were stably transduced cell lines constructed in Example 2, and the cells were digested and counted, the cells were fixed and membrane-broken. The treated cells were then resuspended in a flow cytometry buffer to the cell density of 1×10⁶ cells/ml, and then added into V-bottom 96-well plates at 30 µl/well. Primary antibodies with initial concentration of 10 ug/ml were gradient diluted with the flow cytometry buffer at a three-fold ratio to form 6 gradients. A PBS negative control was set up for each antibody and the positive control antibody was zolbetuximab and diluted under the same condition as above. After inoculation at 4 °C for one hour, the plates were washed once with the flow cytometry buffer. The secondary antibody (abcam, Cat#ab98593) was added at 30 µl/well. After incubation at 4 °C for 30 minutes, the plates were washed twice with the flow cytometry buffer and the cells were shaken dispersedly. The flow cytometry buffer was added at 30 µl/well, and then the cells were tested on the flow cytometer. Original data were entered into the software GraphPad8.0 for graphing and calculation. The results are shown in FIG. 4. The five CLDN18.2 chimeric antigen-binding proteins and the positive control antibody zolbetuximab all bind to mouse CLDN18.2 and cynomolgus monkey CLDN18.2, and their binding activity is gradient-dependent. This indicates that these five chimeric antigen-binding proteins and zolbetuximab not only specifically bind to human CLDN18.2, but also have species cross-binding activity of human, mouse, and cynomolgus monkey.

### Example 8 CDC activity of CLDN18.2 antigen-binding proteins

A cytotoxicity test kit (Promega, Cat#G1780) was used to test the ability of CLDN18.2 antigen-binding proteins to elicit the CDC effect on the SP2/0-human CLDN18.2 cell. The specific process was as follows:
(1) preparation of mediums (A: DMEM+2%FBS+1% double antibody, B: DMEM+2%FBS+1% double antibody+10% rabbit complement) were prepared;
(2) the target cell SP2/0-human CLDN18.2 was centrifuged at 400g for 5 minutes, then resuspended in the above medium A to the target cell density of 4×10⁵ cells/ml, and the suspension was then added into a 96-well cell culture plate at 100 µl/well;
(3) antibodies were diluted with the above medium B into three different concentrations which were 10 µg/ml, 2 µg/ml, and 0.4 µg/ml, respectively; the diluted antibodies were added into wells at 100 µl/well, and two duplicate wells were set up for each concentration point, with zolbetuximab as a positive control antibody and human IgG-Fc as a negative control antibody;
(4) the complement used was Rabbit Complent 3-4 week (Cat#31061-3) purchased from PelFreez Bio Company, and the final concentration of the complement used in the experiment was 5%; and
(5) the control wells were set up according to the requirements of the kit; after the obtained mixture was co-incubated in a 37 °C incubator for 4 hours, the absorbance value at 490 nM was recorded by a microplate reader; the target cell lysis percentage was calculated according to the formula given by the kit, and the data were analyzed and processed with GraphPad prism 8.

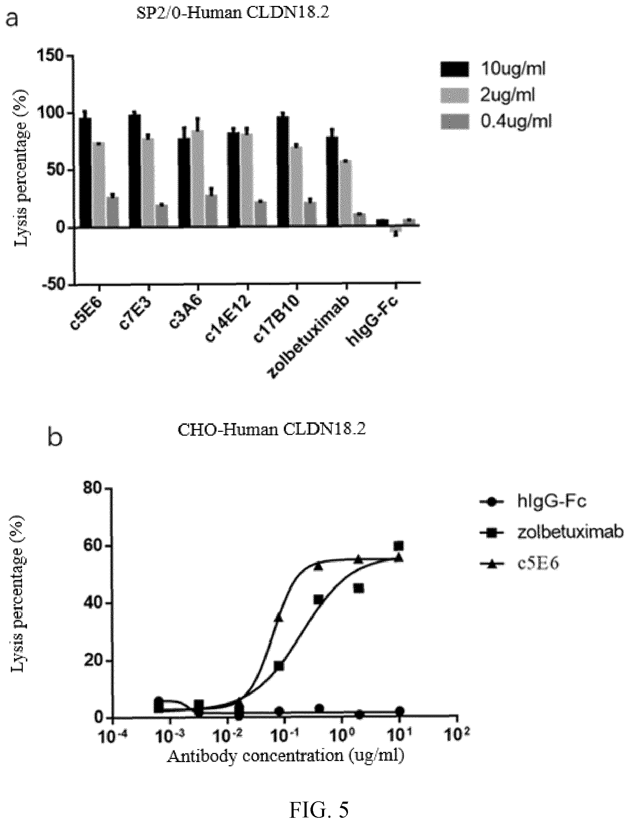

The results are shown in FIG. 5a. In the case where the SP2/0-human CLDN18.2 cell is used as a killing target cell, all tested antigen-binding proteins can elicit a strong CDC effect in a dose-dependent manner, that is, they all have significant CDC activity, and c5E6, c7E3 and c 17B 1 0 among the tested antigen-binding proteins show higher CDC activity than zolbetuximab.

The c5E6 antigen-binding protein was further tested for its ability to elicit the CDC effect on the stable CHO cells with overexpression of human CLDN18.2. Briefly, the CHO-human CLDN18.2 cell was constructed and obtained through our company's lentiviru transfection system, as shown in Example 1. The operation method was the same as above. The cells were loaded in plates at a density of 1.2×10⁴ cells/well. The final concentration of the rabbit complement was 5%, and the antigen-binding proteins were gradient diluted at a five-fold ratio to form 7 concentration gradients, i.e.,10 µg/ml, 2 µg/ml, 0.4 µg/ml, 0.08 µg/ml, 0.016 µg/ml, 0.0032 µg/ml, and 0.64 ng/ml; two duplicate wells were set up for each concentration. Zolbetuximab was used as a positive control antibody, and human IgG-Fc was used as an isotype control antibody. The absorbance value after 4 hours of co-incubation was detected by a microplate reader, and the target cell lysis percentage was calculated. The data were analyzed and processed with GraphPad prism 8.

The results are shown in FIG. 5b. The c5E6 antigen-binding protein can elicit a strong CDC effect on the CHO-human CLDN18.2 cell in a dose-dependent manner and exhibits a higher CDC activity than zolbetuximab.

### Example 9 Analysis of competitive binding activity of CLDN18.2 antigen-binding proteins

The antibodies c5E6 and zolbetuximab were labeled with a biotin (EZ-Link Sulfo-NHS-LC-Biotin, Thermo, A39257), 500 ug for each. The labeling effects of the biotinylated antigen-binding protein molecules were determined by a molecular interaction analyzer OctetRED384 (sartorius). The antibodies were solidified by using a SA sensor. The antibody concentration was 100nM. Labeling was carried out for 180 s. The labeled biotinylated antigen-binding proteins were named Biotinylated-c5E6, Biotinylated-c7E3, Biotinylated-c17B10, and Biotinylated-zolbetuximab.

The epitope competitive binding activity of the above antigen-binding proteins to the CHO-human CLDN18.2 cell (obtained in Example 2) was tested by FACS on the iQue Screener flow cytometer (purchased from IntelliCyt Company) using PBS containing 0.1% BSA as a buffer. The specific process was as follows:
(1) the CHO-huamn CLDN18.2 cell solution with a concentration of 1×10⁶ cells/ml was prepared by using a buffer, and then added into V-bottom 96-well plates (corning 3894) at 30 µl/well;
(2) tested antigen-binding proteins were mixed in a buffer respectively, the Biotinylated-c5E6 antigen-binding protein with a final concentration of 0.5 µg/ml was added in a V-bottom 96-well plate at 30 ul/well, and the Biotinylated-zolbetuximab antigen-binding protein with a final concentration of 5 µg/ml was added in a v-bottom 96-well plate at 30 ul/well;
(3) the competitive antigen-binding proteins (c5E6, c7E3, c17B10 and zolbetuximab) were mixed in a buffer; the final antibody concentration started at 333ug/ml. The antibodies were gradient diluted at a 4-fold ratio to form 10 concentration gradients; wells with PBS were set up as negative control wells and the prepared antibodies of different concentrations were added, at 30 µl/well into the V-bottom 96-well plates loaded with the target cells and test antibodies, and then mixed well;
(4) incubation was carried out for one hour in a 4 °C refrigerator;
(5) The buffer was added at 110 µl/well, the cell solution was centrifuged at 500 g for 5 minutes, the supernatant was discarded, and the cells were shaken dispersedly;
(6) step (5) was performed again;
(7) the fluorescent secondary antibody (BD phamingen APC Streptavidin, Cat#554067) was mixed in the buffer at a ratio of 1:500, and the antibody solution was added to the cell at 30 µl/well and mixed well, and the cells were incubated in a 4 °C refrigerator for 30 minutes;
(8) the buffer was added at 170 µl/well, the solution was centrifuged at 500 g for 5 minutes, the supernatant was discarded, and the cells were shaken dispersedly;
(9) step (8) was performed again; and
(10) the buffer centrifuged at 25 µl/well, the solution was mixed well, and then FACS was carried out by a flow cytometer.

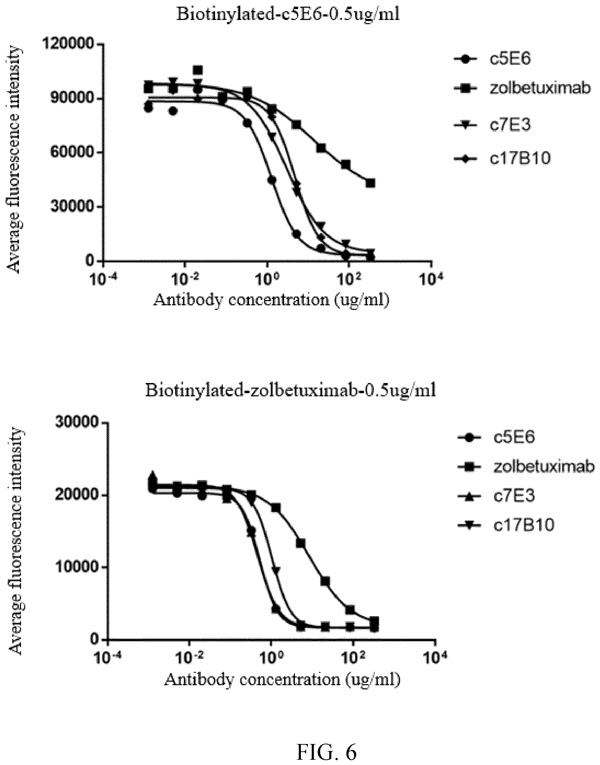

The FACS results of the epitope competitive binding activity are shown in FIG. 6. The isolated antigen-binding proteins c17B10/c7E3 of the present application both compete against the Biotinylated-c5E6, and the antigen-binding proteins c17B10/c7E3 completely compete against the Biotinylated-c5E6 for the binding to cellular antigens at a high concentration (333 ug/ml). It is speculated that they have the same antigen-binding epitope. In contrast, there is weak competition between zolbetuximab and Biotinylated-c5E6. As the concentration of the antibody zolbetuximab increases, the Median signal value decreases to some extent and shows a certain concentration dependence. However, when the concentration of the antibody zolbetuximab increases to 333 ug/ml, its competitive activity and strength are still far weaker than those of other antigen-binding proteins, and it cannot completely compete against the Biotinylated-c5E6 antigen-binding protein. It is speculated that the antigen-binding epitopes of zolbetuximab and c5E6 are not completely consistent, showing crossover or inclusion (the epitope of the antibody zolbetuximab is included in c5E6) relationship.

### Example 10 In vivo anti-tumor activity of CLDN18.2 antigen-binding proteins

The antigen-binding proteins (c5E6, c7E3, c17B10) prepared in Example 5, the positive control antibody zolbetuximab and the isotype control antibody human IgG-Fc were selected to test their anti-tumor activity in C57BL/6 mice inoculated with a mouse colon cancer cell. The above six antigen-binding proteins were purified and obtained using our company's Expi293 transient transfection system, and the endotoxin of the antigen-binding proteins was controlled at 4 EU/mg or below. The operation was specifically implemented as follows:

115 female C57BL/6 mice were subcutaneously inoculated with the MC38-human CLDN18.2 cell in the right abdomen at a cell volume of 1.1 × 10⁶. Seven days after inoculation, 80 mice with a tumor volume within a range of 31.33 mm³ to 116.31 mm³ (average tumor volume of 71.59 mm³) were selected and divided into 10 groups according to tumor volume, with 8 mice in each group, and the day of random grouping of mice was defined as Day 0. After grouping, the mice were administered to the tail vein once a week for four consecutive weeks in two doses of 30 mg/kg and 7.5 mg/kg. The grouping and dosage regimen of mice are shown in Table 4. The body weight and tumor volume of mice were measured three times a week during the dosing period, and the tumor volume was calculated according to the formula TV = (length × width²) / 2, and then the tumor growth inhibition rate (TGI) and T/C value were calculated according to the tumor volume. When the tumor volume reached 2000 mm³, the experiment was stopped and the mice were euthanized.

**Table 4. Grouping and dosing regimen of in vivo efficacy experiments of CLDN18.2 antigen-binding proteins**

| Mouse group | Antibody administrated | Dose (mg/kg) | Number of mice | Administration route and time |
|---|---|---|---|---|
| 1 | Phosphate buffer | 10 ml/kg | 8 | tail vein, once a week x 4 weeks |
| 2 | hIgG-Fc | 7.5 mg/kg | 8 | tail vein, once a week x 4 weeks |
| 3 | zolbetuximab | 7.5 mg/kg | 8 | tail vein, once a week x 4 weeks |
| 4 | zolbetuximab | 30 mg/kg | 8 | tail vein, once a week x 4 weeks |
| 5 | c5E6 | 7.5 mg/kg | 8 | tail vein, once a week x 4 weeks |
| 6 | c5E6 | 30 mg/kg | 8 | tail vein, once a week x 4 weeks |
| 7 | c17B10 | 7.5 mg/kg | 8 | tail vein, once a week x 4 weeks |
| 8 | c17B10 | 30 mg/kg | 8 | tail vein, once a week x 4 weeks |
| 9 | c7E3 | 7.5 mg/kg | 8 | tail vein, once a week x 4 weeks |
| 10 | c7E3 | 30 mg/kg | 8 | tail vein, once a week x 4 weeks |

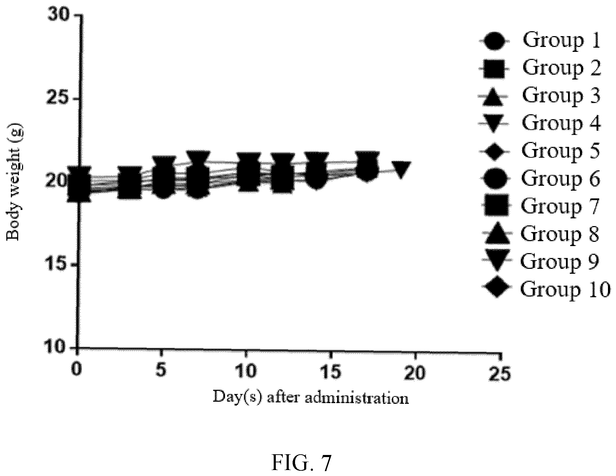

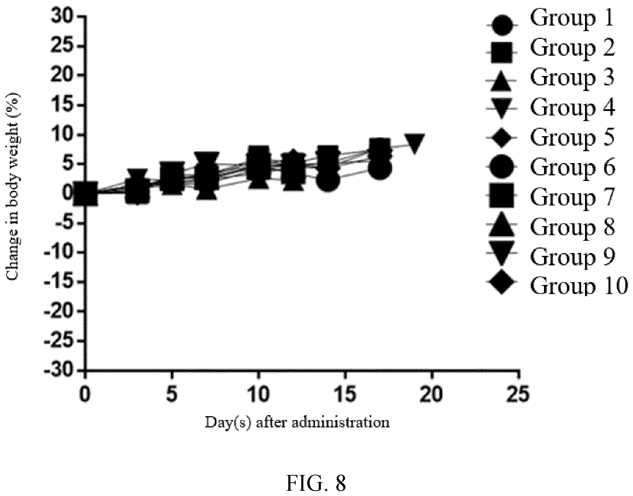

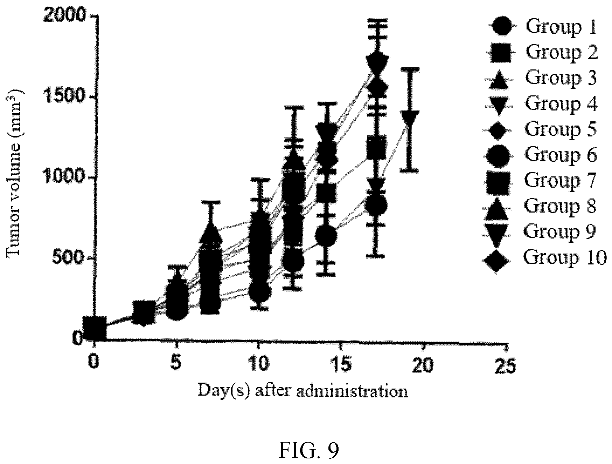

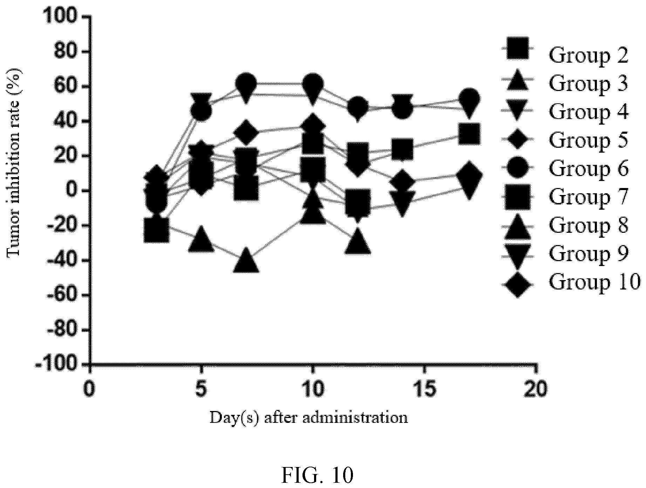

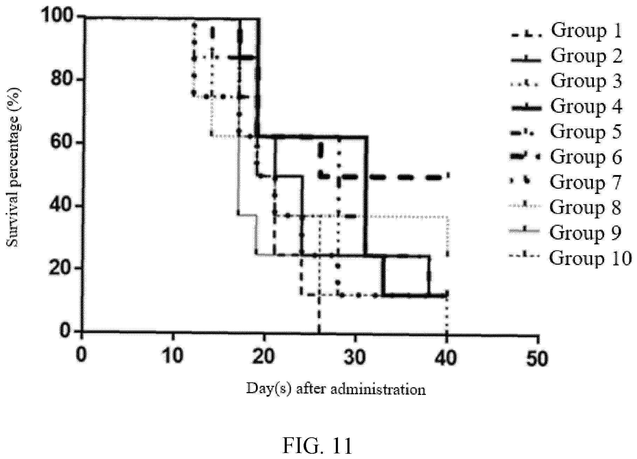

By using a subcutaneous mouse colon cancer model, the anti-tumor effects of zolbetuximab, c17B10, c5E6 and c7E3 administered via the tail vein route were evaluated in female C57BL/6 mice. The results showed that in MC38-human CLDN18.2 tumor-bearing C57BL/6 mice, all the administrated mice well tolerate the antibodies, the mice keep stable in body weight and have no any adverse reactions. The change in body weight of mice and relative change (%) are shown in FIGS. 7 and 8. By analyzing the tumor volume change curves and tumor growth inhibition curves before Day 17, compared with the PBS control group, the c5E6 and zolbetuximab high-dose administration groups show better anti-tumor effects, and the anti-tumor effects show significant differences (**** , P<0.0001), as shown in FIGS. 9 and 10. By analyzing the Kaplan-Meier survival curves before Day 40, compared with the PBS control group, the c5E6 low-dose, c5E6 high-dose and zolbetuximab high-dose administration groups all show high mouse survival percentages, and there were significant differences (*, P <0.05), and among them, the antigen-binding protein c5E6 has the highest anti-tumor activity. The results are shown in FIG. 11.

### Example 11 Production of CLDN18.2 single-chain antibodies and identification of antigen-binding activity

In this example, lentivirus infection and FACS were carried out to obtain a cell that stably expresses human CLDN18.2, namely the SP2/0-hCLDN18.2 cell. Cellular immunization was performed on Balb/c mice. The immunogen was the SP2/0-hCLDN18.2. Mouse hybridoma technology, FACS and subcloning were carried out to obtain a CLDN18.2-positive monoclonal cell. The IgG-like murine CLDN18.2 monoclonal antibodies were cultured in vitro. The degenerate primer amplification method was performed for sequencing to obtain the sequences of a heavy chain variable region (V_{H}) and a light chain variable region (V_{L}), and the heavy chain variable region (V_{H}) and light chain variable region (V_{L}) of the antibody were connected into a recombinant gene through a synthetic linker peptide (Linker) gene. The antibody expressed by the recombinant gene was a CLDN18.2 single-chain antibody, i.e., 5E6-scFv (SEQ ID NO: 90). In the meanwhile, lentivirus infection and FACS were carried out to obtain the SP2/0-hCLDN18.1 cell that stably expresses human CLDN18.1.

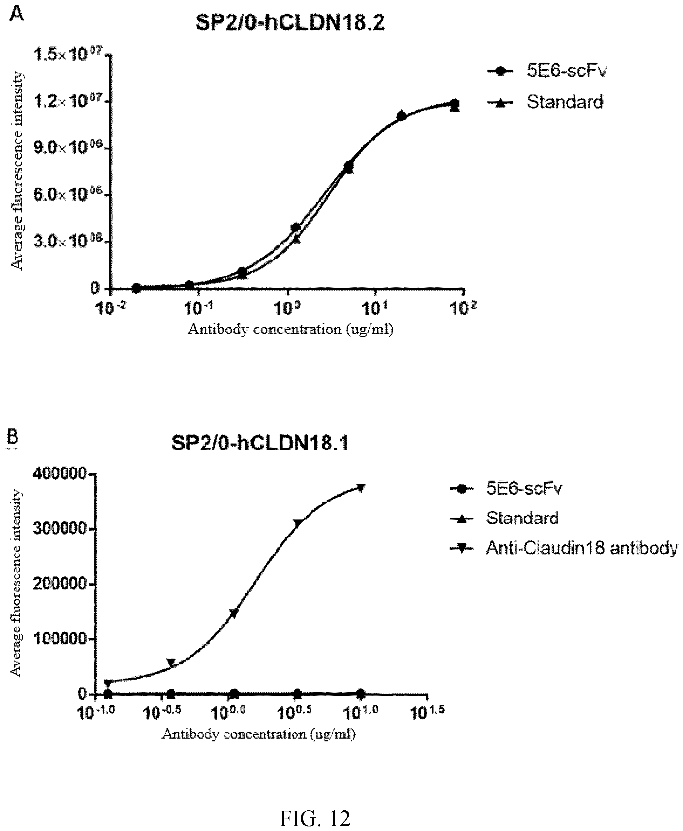

The specific binding activity of the single-chain antibody 5E6-scFv and the Standard antibody (the VH comprising an amino acid sequence as set forth in SEQ ID NO: 92 and the VL comprising an amino acid sequence as set forth in SEQ ID NO: 93) was identified by FACS as follows. After counting, the SP2/0-hCLDN18.2 cell was resuspended in a flow cytometry buffer, adjusted to the density of 1×10⁶ cells/ml, and then added into V-bottom 96-well plates at 30 µl/well. Primary antibodies with initial working concentration of 80 µg/ml were added at 30 µl/well, and then gradient diluted with the flow cytometry buffer at a four-fold ratio to form 7 gradients. APBS negative control was set up for each antibody. After inoculation at 4 °C for one hour, the plates were washed once with the flow cytometry buffer. The secondary antibody, i.e., goat F(ab')2 anti-human IgG-Fc (DyLight^{®} 650) (abcam, Cat#ab98593), was added at 30 µl/well. After inoculation at 4 °C for 30 minutes, the plates were washed twice with the flow cytometry buffer and the cells were shaken dispersedly. The flow cytometry buffer was added at 25 µl/well, and then the cells were ready for FACS on the flow cytometer. In the meanwhile, after counting, the SP2/0-hCLDN18.1 cell was loaded in plates. Primary antibodies with initial working concentration of 10 µg/ml were gradient diluted at a three-fold ratio to form 5 concentration gradients in total. The secondary antibody used was goat F(ab')2 anti-human IgG-Fc (DyLight^{®} 650) (abcam, Cat#ab98593). Original data were entered into the software GraphPad8.0 for graphing and calculation. The results are shown in FIG. 12: like the Standard antibody, the 5E6-scFv single-chain antibody can specifically bind to human CLDN18.2.

### Example 12 Identification of binding activity of CLDN18.2 single-chain antibodies to non-target cells

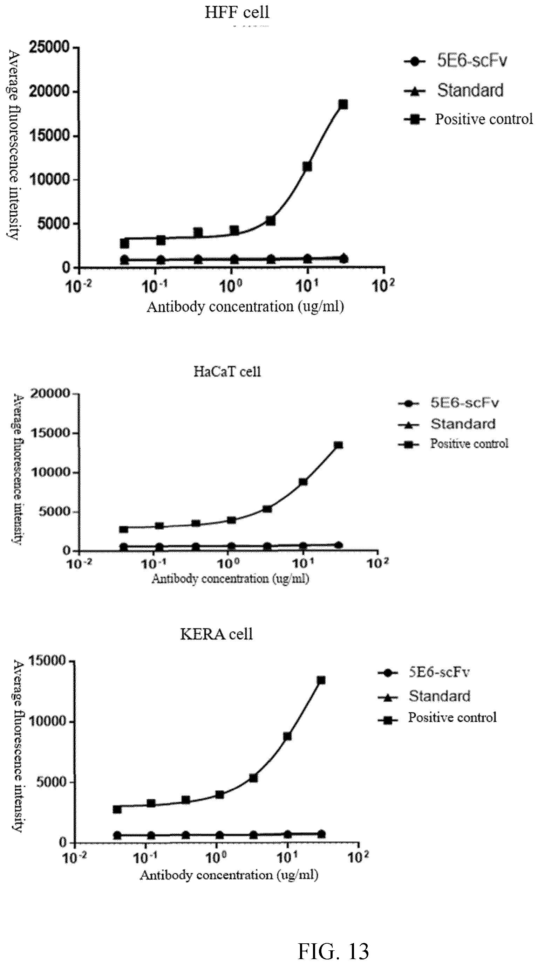

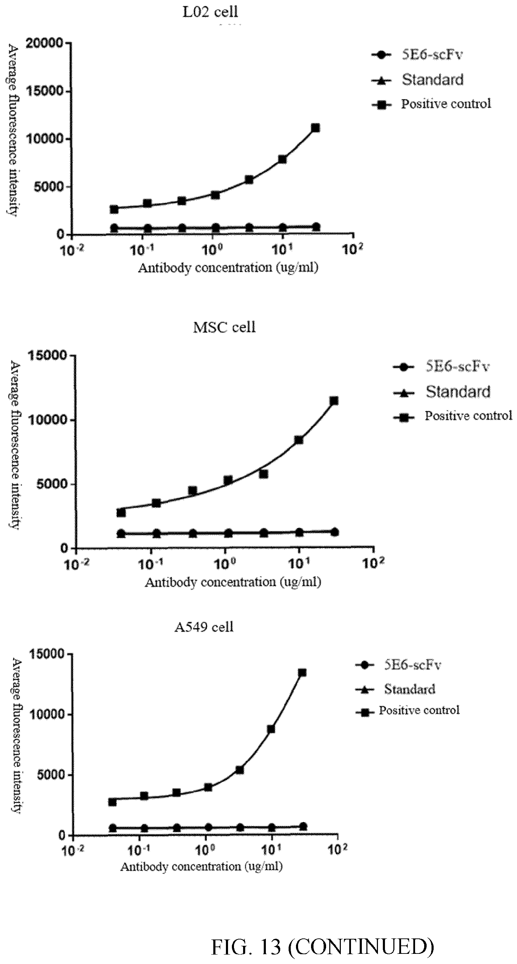

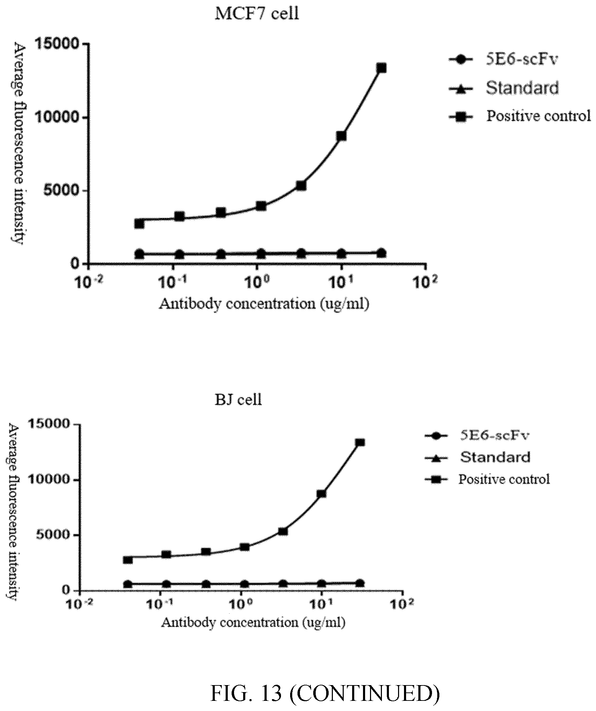

In this example, FACS was carried out to identify the binding activity of the single-chain antibody 5E6-scFv to various non-target cells in human tissues. The cells were human foreskin fibroblast (HFF), human immortalized epidermal cell (HaCaT), human normal liver cell (LO2), mesenchymal stem cell (MSC), human normal epidermal cell (KERA), adenocarcinoma human alveolar basal epithelium cell (A549), human breast cancer cell (MCF-7), and human skin fibroblast (BJ). The antibody with an initial working concentration of 30 µg/ml was gradient diluted at a three-fold ratio to form 7 concentration gradients in total. The secondary antibody used was goat F(ab')2 anti-human IgG-Fc (DyLight^{®} 650) (abcam, Cat#ab98593). After incubation, the cell solution was centrifuged and then resuspended in the flow cytometry buffer, and then tested on the flow cytometer. The results are entered into the software GraphPad8.0 for graphing. The results are shown in FIG. 13. Like the Standard antibody, the single-chain antibody 5E6-scFv does not bind non-specifically to various non-target cells in human tissues, and its specificity for tumor targets has been preliminarily verified, that is, there is no "off-target effect" phenomenon.

### Example 13 Anti-tumor activity of CLDN18.2 antigen-binding proteins in mouse model of human gastric cancer

As stated in Example 10, the human CLDN18.2 antigen-binding protein (c5E6), the positive control antibody zolbetuximab and the isotype control antibody human IgG-Fc were selected to test their anti-tumor activity in female nude mice inoculated with a human gastric cancer cell (NUGC-4). The operation was specifically implemented as follows:
28 female nude mice were subcutaneously inoculated with the NUGC-4 cell at a cell volume of 3 × 10⁶. Seven days after inoculation, 20 mice with an average tumor volume of 80 mm³ were selected and divided equally into 4 groups according to tumor volume, with 5 mice in each group, and the day of random grouping of mice was defined as Day 0. After grouping, the mice were administered twice a week for three consecutive weeks. The grouping and dosage regimen of mice are shown in Table 5. The body weight and tumor volume of mice were measured three times a week during the dosing period, and the tumor volume was calculated according to the formula TV = (length × width²) / 2. When the tumor volume reached 3000 mm³, the experiment was stopped and the mice were euthanized.

**Table 5. Grouping and dosing regimen of in vivo efficacy experiments in mouse models of human gastric cancer**

| Mouse group | Antibody administrated | Dose | Number of mice | Administration route and time |
|---|---|---|---|---|
| G1 | PBS | 10 ml/kg | 5 | Intraperitoneal administration, twice a week x 3 weeks |
| G2 | hIgG-Fc | 10mg/kg | 5 | Intraperitoneal administration, twice a week x 3 weeks |
| G3 | zolbetuximab | 10 mg/kg | 5 | Intraperitoneal administration, twice a week x 3 weeks |
| G4 | c5E6 | 10 mg/kg | 5 | Intraperitoneal administration, twice a week x 3 weeks |

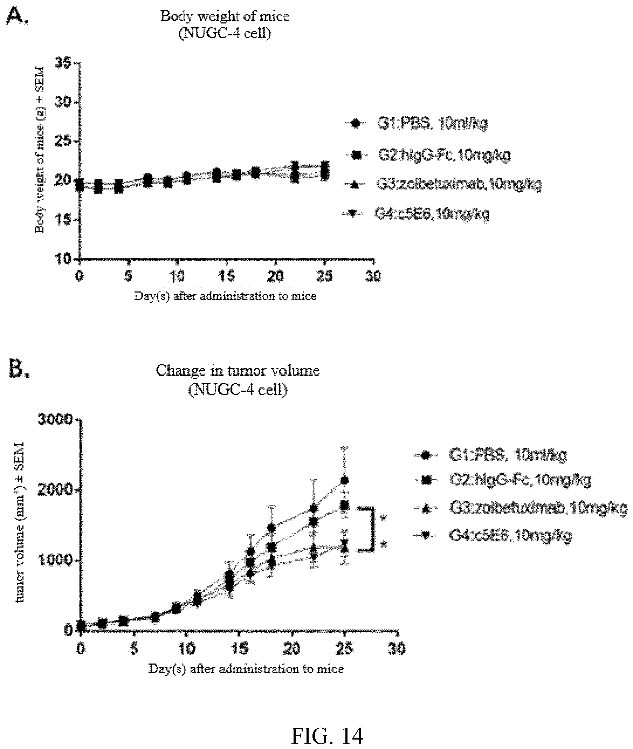

The subcutaneous NUGC-4 human gastric cancer tumor model was used to evaluate the anti-tumor effects of intraperitoneal administration of antibodies zolbetuximab and c5E6 in female nude mice. The experiment was observed until 25 days after administration. The results show that in NUGC-4 tumor-bearing female nude mice, all the administered mice well tolerate the antibodies, and the mice keep stable in body weight and have no any adverse reactions. The change in body weight of mice is shown in FIG. 14A. During the observation period, the tumor volume change curves are shown in FIG. 14B. The mice in the group G3: zolbetuximab, 10 mg/kg and the group G4: c5E6, 10 mg/kg all show a certain anti-tumor activity relative to the isotype control group G2, and both groups have statistically significant differences (p<0.05); there is no significant difference in the anti-tumor activity between G3 and G4, and G3 and G4 have similar anti-tumor levels.

### Example 14 Stable expression of CLDN18.2-specific CARs in T cell

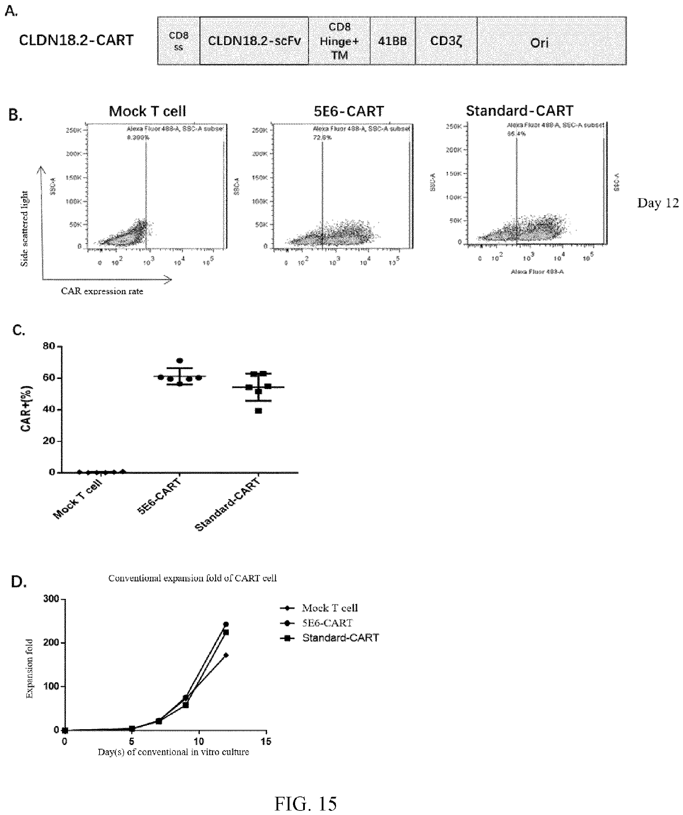

As shown in FIG. 15A, the CLDN18.2-specific CAR structure in this example was composed of a human CD8 signal peptide (SEQ ID NO: 88), an anti-human CLDN18.2 single-chain antibody (SEQ ID NO: 90), a human CD8 hinge region (SEQ ID NO: 83), a human CD8 transmembrane domain (SEQ ID NO: 84), a human 4-1BB intracellular costimulatory domain (SEQ ID NO: 85), a human CD3ζ intracellular activation domain (SEQ ID NO: 86) and an added Ori new element (SEQ ID NO: 91). First, this example explored the expression of the CLDN18.2-specific CAR in human T cell and the expansion fold of CART cell under conventional in vitro culture conditions. The specific method was as follows:
1) The human PBMC cell frozen in liquid nitrogen was resuscitated in a 37 °C water bath and then centrifuged, resuspended and rinsed with a 11ml PBS+1ml PBMC system three times (500g, 5min; 400g, 5min; 300g, 5min). The rinsed human PBMC cell was mixed with CD3 MicroBeads, human (Miltenyi Biotec, 130-050-101) and CD3 positive selection was carried out by using a magnetic stand, that is, CD3⁺ T cells was separated and retained. CD3⁺T cells were resuspended in a culture medium containing 4%FBS+X-VIVO (Lonza Company) +20ng/ml cytokine 1+10ng/ml cytokine 2 to a cell density of 1×10⁶ cells/ml. CD3/CD28 magnetic beads (Thermo Fisher 40203D, which were washed twice with the culture medium and held with the magnetic strand still for 1 minute) were added at a ratio of 1:3 (cells: magnetic beads) to activate T cell. T cell was added to the magnetic beads and mixed well, and the culture medium was added to each well of the 12-well plate to 700 µl, with the count of cells being 7×10⁵ cells/well and at the density of 1×10⁶ cells/ml. The day of sorting was defined as Day 0.
2) After the activated T cell was held still and incubated in a 37 °C CO₂ incubator for 20 hours, the corresponding virus supernatant was added at a ratio of virus multiplicity of infection (MOI) being 4, and polybrene was then added to reach a final concentration of 10 µg/ml. The mixed solution was pipetted and mixed well and then centrifuged by a horizontal centrifuge at 1200 rpm for 1 hour. The well plate was placed back into the 37°C CO₂ incubator for incubation for 24 hours. The day of infection was defined as Day 1.
3) 24 hours later, the cells in each well of the 12-well plate were repeatedly pipetted, transferred to a 1.5 ml EP tube, and centrifuged at 400g for 5 minutes. The supernatant was removed. The cells were resuspended in 1 ml of fresh X-VIVO complete medium to the cell density of 7× 10⁵ cells/ml and then incubated in a 37°C CO₂ incubator. If the medium turns yellow, more medium should be added. The cells were counted every 2 days, and fresh X-VIVO complete medium was added to adjust the cell density back to 7× 10⁵ cells/ml. The statistical counting results were recorded, and the software GraphPad8.0 was used for graphing and for calculating the expansion fold of CART cell under conventional culture conditions.
4) Detection of cell positivity rate of CAR-T cell cultured for 9-14 days: The virus used to infect the cells carried Myc-tag, so after the cells were infected with the virus, the Myc positivity rate was tested by FACS to obtain the CAR expression positivity rate. The direct-labeled detection antibody used was Myc-Tag (9B11) Mouse mAb (Alexa Fluor^{®} 488 Conjugate) (Cell Signaling, 2279S).

The cells were respectively infected with human CLDN18.2 sequence viruses, including 5E6 sequence virus and Standard sequence virus, at the MOI of 4. The results are shown in FIG. 15B. On day 12 of in vitro culture of the CART cell, the CAR positivity rate was tested by FACS. The positivity rate of 5E6-CART was 72.6%, and the positivity rate of Positive control CAR-T was 65.4%. In this example, a total of 6 PBMC donars were adopted for positivity rate verification. FIG. 15C shows that the positivity rate of 5E6-CART in 6 different PBMC donar remains at around 60%, just likePositive control CAR-T. FIG. 15D shows that the cumulative expansion fold of 5E6-CART cells conventionally cultured in vitro for 12 days is about 240, which is not significantly different from the expansion fold of Standard-CART.

### Example 15 CLDN18.2-specific CAR can specifically kill target cells in vitro

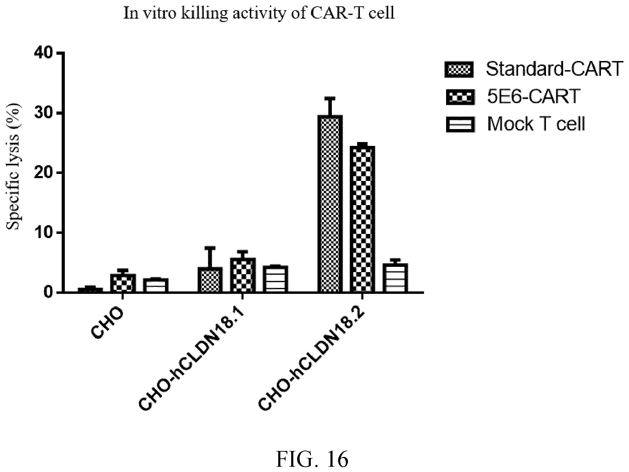

In this example, lentivirus infection and FACS were carried out to obtain a CHO-hCLDN18.2 cell that stably highly expresses human CLDN18.2 and a CHO-hCLDN18.1 cell that stably highly expresses human CLDN18.1. In this example, a cytotoxicity test kit (Promega, Cat#G1780) was further used to evaluate the specific killing ability of CAR-T cell in vitro by a LDH method. The operation steps were as follows:
The CAR-T cell and Mock T cell that had been conventionally cultured for 9 days in Example 14 were centrifuged separately and then resuspended in a blank X-VIVO medium to the cell density of 1×10⁵ cells/ml. Used three target cells were CHO, CHO-hCLDN18.2, and CHO-hCLDN18.1, respectively. The three target cells were separately digested and counted and then resuspended in the blank X-VIVO medium to the density of 5×10⁵ cells/ml. The target cell suspension and the CAR-T/Mock T cell suspension were mixed by a volume system and added to V-bottom 96-well plates, with 100 ul of target cell suspension and 100 ul of CAR-T/Mock T cell suspension in each well. The control wells were set up according to the requirements of the kit. After the obtained mixture was incubated in a 37 °C incubator for 24 hours, the absorbance value at 490nM was recorded by a microplate reader. The target cell lysis percentage was calculated according to the formula given by the kit, and the data were analyzed and processed with GraphPad prism 8. The results are shown in FIG. 16. 5E6-CART specifically induces the lysis of CLDN18.2-positive target cells in vitro, but has no lysis effect on negative and CLDN18.1-positive cells, just like Standard-CART. It indicates that 5E6-CART cell has in vitro high-specificity killing activity for human CLDN18.2 target cells.

### Example 16 Cytokines secretion of CLDN18.2-specific CAR

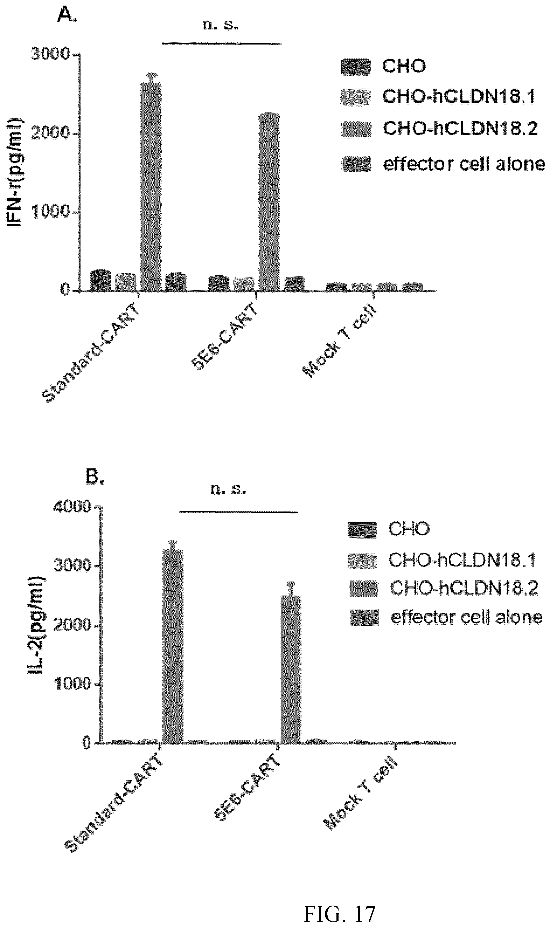

In this example, Human IFN-γ ELISA kit (R&D, DY285B) and Human IL-2 ELISA kit (R&D, DY202) were respectively used to analyze the secretion of IFN-γ and IL-2 by CAR-T during the process of killing target cells. Specifically, the target cell (CHO-hCLDN18.2) with high expression of CLDN18.2, the control cell (CHO-hCLDN18.1) with high expression of CLDN18.1 and the negative cell CHO were respectively inoculated into a sterile 96-well plate at a cell volume of 1×10⁴ cells per well, and effector cells such as CAR-T cell (5E6-CART and Positive control CAR-T) and unmodified T cell (Mock T cell) were added into the target cell at a ratio of Effector: Target being 5:1. After incubation for 24 hours, operations were performed according to the instructions of the kit, and the supernatant was taken to detect the contents of IL-2 and IFN-γ by using enzyme-linked immunoassay (ELISA). The results are shown in FIGS. 17A and 17B. 5E6-CART secreted a high cytokines level when co-incubated with the CLDN18.2-positive cell, but had no significant cytokines secretion when co-incubated with the negative cell and the CLDN18.1-positive cell, just like the Standard-CART. Therefore, the 5E6-CART cell has a specific cytokine-inducing effect on the tumor cell with high expression of CLDN18.2.

### Example 17 Anti-tumor effect of CLDN18.2-specific CAR-T cell in a mouse colon cancer CDX model

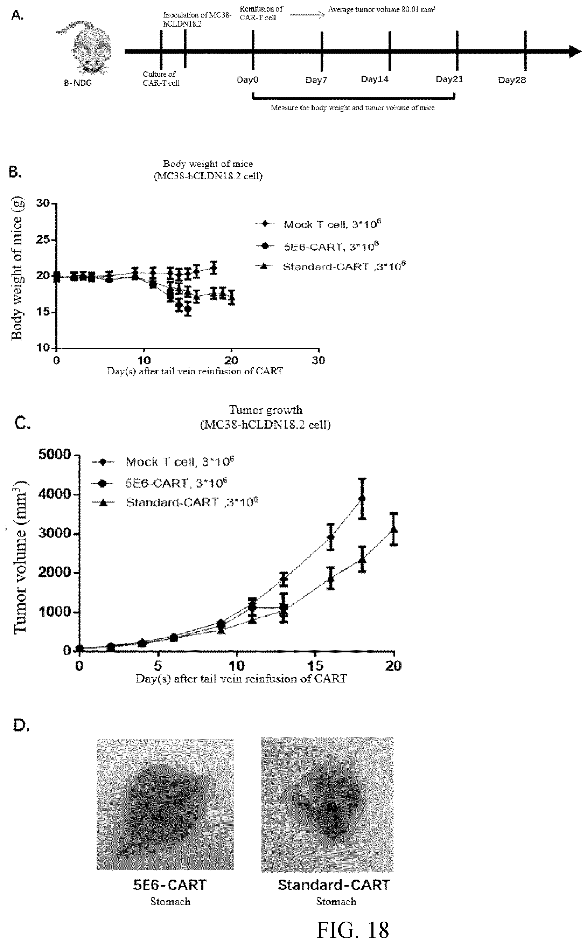

In this example, the human CLDN18.2 gene was first introduced into the MC38 cell in a lentivirus manner, and then the mouse colon cancer cell with high expression of human CLDN18.2, i.e., MC38-hCLDN18.2 cell, was sorted by a flow cytometer. Female B-NDG severely immunodeficient mice were used to construct a mouse colon cancer tumor model through subcutaneous injection to verify the anti-tumor effect of the CLDN18.2-specific CAR-T cell in mice. The MC38-hCLDN18.2 cell was inoculated at a dose of 1.5×10⁶ cells/mouse. Eight days after the inoculation, mice with a tumor volume within a range of 41.68 mm³ to 120.6 mm³ (average tumor volume of 80.01 mm³) were randomly divided into 3 groups, 8 mice in each group, and the CAR-T (or Mock T) cell that had been cultured for 10 days was reinfused into the tail vein. The CAR-T reinfusion dose of 5E6-CART and Standard-CART groups was 3×10⁶ cells/mouse, and the reinfusion dose of the blank control Mock T cell group was 1×10⁷ cells/mouse. The tumor volume and body weight of the mice were measured three times a week and observed for 4 weeks, as shown in FIG. 18A. After reinfusion of the CAR-T (or Mock T) cell into the tail vein of mice, for the 5E6-CART group, the mice lost more than 10% of their body weight on D13, the relative change in the body weight of a single mouse was -34.51% on D15, and the first death of mice appeared on D13; for the Standard-CART group, the mice lost more than 10% of their body weight on D16, the relative change in the body weight of a single mouse was -36.93% on D16, and the first death of mice appeared on D18. Due to severe weight loss in mice, the experiment ended on PG-D22. No obvious anti-tumor effect was observed at the end of the experiment, as shown in FIGS. 18B and 18C. After the mice were euthanized and dissected, it was found that the mice in both the 5E6-CART group and the Standard-CART group had abnormal gastric damage and bleeding, as shown in FIG. 18D. In summary, the mice in the 5E6-CART group showed intolerance problems and obvious side effects in this tumor model, which were specifically manifested as weight loss, back hair standing on end, curling up, and stomach bleeding; the mice in the Mock T group showed tolerance and their body weight was normal.

## Claims

1. An isolated antigen-binding protein, comprising an HCDR3, wherein said HCDR3 comprises an amino acid sequence as set forth in SEQ ID NO: 67.

2. The isolated antigen-binding protein of claim 1, wherein said HCDR3 comprises an amino acid sequence as set forth in any one of SEQ ID NO: 4, SEQ ID NO: 12, and SEQ ID NO: 18.

3. The isolated antigen-binding protein of any one of claims 1-2, comprising an HCDR2, wherein said HCDR2 comprises an amino acid sequence as set forth in SEQ ID NO: 68 or SEQ ID NO: 11.

4. The isolated antigen-binding protein of claim 3, wherein said HCDR2 comprises an amino acid sequence as set forth in SEQ ID NO: 3 or SEQ ID NO: 17.

5. The isolated antigen-binding protein of any one of claims 1-4, comprising an HCDR1, wherein said HCDR1 comprises an amino acid sequence as set forth in any one of SEQ ID NO: 2, SEQ ID NO: 10, and SEQ ID NO: 16.

6. The isolated antigen-binding protein of claim 5, comprising an H-FR1, wherein a C terminal of said H-FR1 is directly or indirectly linked to an N terminal of said HCDR1, and said H-FR1 comprises an amino acid sequence as set forth in SEQ ID NO: 72.

7. The isolated antigen-binding protein of claim 6, wherein said H-FR1 comprises an amino acid sequence as set forth in any one of SEQ ID NO: 34, SEQ ID NO: 43, and SEQ ID NO: 49.

8. The isolated antigen-binding protein of any one of claims 5-7, comprising an H-FR2, wherein said H-FR2 is located between said HCDR1 and said HCDR2 and comprises an amino acid sequence as set forth in SEQ ID NO: 73.

9. The isolated antigen-binding protein of claim 8, wherein said H-FR2 comprises an amino acid sequence as set forth in SEQ ID NO: 35 or SEQ ID NO: 44.

10. The isolated antigen-binding protein of any one of claims 3-9, comprising an H-FR3, wherein said H-FR3 is located between said HCDR2 and said HCDR3 and comprises an amino acid sequence as set forth in SEQ ID NO: 80 or SEQ ID NO: 45.

11. The isolated antigen-binding protein of claim 10, wherein said H-FR3 comprises an amino acid sequence as set forth in SEQ ID NO: 36 or SEQ ID NO: 42.

12. The isolated antigen-binding protein of any one of claims 1-11, comprising an H-FR4, wherein an N terminal of said H-FR4 is linked to a C terminal of said HCDR3, and said H-FR4 comprises an amino acid sequence as set forth in SEQ ID NO: 74.

13. The isolated antigen-binding protein of claim 12, wherein said H-FR4 comprises an amino acid sequence as set forth in SEQ ID NO: 46 or SEQ ID NO: 37.

14. The isolated antigen-binding protein of any one of claims 1-13, comprising a VH, wherein said VH comprises an amino acid sequence as set forth in SEQ ID NO: 78.

15. The isolated antigen-binding protein of claim 14, wherein said VH comprises an amino acid sequence as set forth in any one of SEQ ID NO: 1, SEQ ID NO: 9, SEQ ID NO: 15, and SEQ ID NO: 19.

16. The isolated antigen-binding protein of any one of claims 1-15, comprising an LCDR3, wherein said LCDR3 comprises an amino acid sequence as set forth in SEQ ID NO: 69.

17. The isolated antigen-binding protein of claim 16, wherein said LCDR3 comprises an amino acid sequence as set forth in any one of SEQ ID NO: 8, SEQ ID NO: 14, SEQ ID NO: 23, and SEQ ID NO: 25.

18. The isolated antigen-binding protein of any one of claims 1-17, comprising an LCDR2, wherein said LCDR2 comprises an amino acid sequence as set forth in SEQ ID NO: 70.

19. The isolated antigen-binding protein of claim 18, wherein said LCDR2 comprises an amino acid sequence as set forth in SEQ ID NO: 7 or SEQ ID NO: 22.

20. The isolated antigen-binding protein of any one of claims 1-19, comprising an LCDR1, wherein said LCDR1 comprises an amino acid sequence as set forth in SEQ ID NO: 71.

21. The isolated antigen-binding protein of claim 20, wherein said LCDR1 comprises an amino acid sequence as set forth in SEQ ID NO: 6 or SEQ ID NO: 21.

22. The isolated antigen-binding protein of any one of claims 20-21, comprising an L-FR1, wherein a C terminal of said L-FR1 is directly or indirectly linked to an N terminal of said LCDR1, and said L-FR1 comprises an amino acid sequence as set forth in SEQ ID NO: 75.

23. The isolated antigen-binding protein of claim 22, wherein said L-FR1 comprises an amino acid sequence as set forth in any one of SEQ ID NO: 38, SEQ ID NO: 47, SEQ ID NO: 50 and SEQ ID NO: 53.

24. The isolated antigen-binding protein of any one of claims 20-23, comprising an L-FR2, wherein said L-FR2 is located between said LCDR1 and said LCDR2 and comprises an amino acid sequence as set forth in SEQ ID NO: 39.

25. The isolated antigen-binding protein of any one of claims 18-24, comprising an L-FR3, wherein said L-FR3 is located between said LCDR2 and said LCDR3 and comprises an amino acid sequence as set forth in SEQ ID NO: 76.

26. The isolated antigen-binding protein of claim 25, wherein said L-FR3 comprises an amino acid sequence as set forth in any one of SEQ ID NO: 40, SEQ ID NO: 48, and SEQ ID NO: 51.

27. The isolated antigen-binding protein of any one of claims 16-26, comprising an L-FR4, wherein an N terminal of said L-FR4 is linked to a C terminal of the LCDR3, and said L-FR4 comprises an amino acid sequence as set forth in SEQ ID NO: 77.

28. The isolated antigen-binding protein of claim 27, wherein said L-FR4 comprises an amino acid sequence as set forth in any one of SEQ ID NO: 41, SEQ ID NO: 52, and SEQ ID NO: 54.

29. The isolated antigen-binding protein of any one of claims 1-28, comprising a VL, wherein said VL comprises an amino acid sequence as set forth in SEQ ID NO: 79.

30. The isolated antigen-binding protein of claim 29, wherein said VL comprises an amino acid sequence as set forth in any one of SEQ ID NO: 5, SEQ ID NO: 13, SEQ ID NO: 20, and SEQ ID NO: 24.

31. The isolated antigen-binding protein of any one of claims 1-30, comprising any set of amino acid sequences selected from the groups consisting of:
1) said VH comprising the amino acid sequence as set forth in SEQ ID NO: 1 and said VL comprising the amino acid sequence as set forth in SEQ ID NO: 5;
2) said VH comprising the amino acid sequence as set forth in SEQ ID NO: 15 and said VL comprising the amino acid sequence as set forth in SEQ ID NO: 5;
3) said VH comprising the amino acid sequence as set forth in SEQ ID NO: 9 and said VL comprising the amino acid sequence as set forth in SEQ ID NO: 13;
4) said VH comprising the amino acid sequence as set forth in SEQ ID NO: 19 and said VL comprising the amino acid sequence as set forth in SEQ ID NO: 20; and
5) said VH comprising the amino acid sequence as set forth in SEQ ID NO: 19 and said VL comprising the amino acid sequence as set forth in SEQ ID NO: 24.

32. The isolated antigen-binding protein of any one of claims 1-31, comprising an antibody heavy chain constant region.

33. The isolated antigen-binding protein of claim 32, wherein said antibody heavy chain constant region is derived from a human IgG heavy chain constant region.

34. The isolated antigen-binding protein of any one of claims 32-33, wherein said antibody heavy chain constant region is derived from a human IgG1 heavy chain constant region.

35. The isolated antigen-binding protein of any one of claims 1-34, comprising an antibody light chain constant region.

36. The isolated antigen-binding protein of claim 35, wherein said antibody light chain constant region is derived from a human Igκ constant region.

37. The isolated antigen-binding protein of any one of claims 1-36, comprising an antibody or an antigen-binding fragment thereof.

38. The isolated antigen-binding protein of claim 37, wherein said antigen-binding fragment comprises Fab, Fab', Fv fragment, F(ab')₂, F(ab)₂, scFv, di-scFv, and/or dAb.

39. The isolated antigen-binding protein of any one of claims 37-38, wherein said antibody is selected from one or more groups consisting of a monoclonal antibody, a chimeric antibody, a humanized antibody, and a fully human antibody.

40. The isolated antigen-binding protein of any one of claims 1-39, substantially not competing with a reference antibody for binding to CLDN18.2, as verified in FACS, wherein said reference antibody comprises a heavy chain variable region (VH) and a light chain variable region (VL), the VH of said reference antibody comprises an amino acid sequence as set forth in SEQ ID NO: 55, and the VL of said reference antibody comprises an amino acid sequence as set forth in SEQ ID NO: 56.

41. The isolated antigen-binding protein of any one of claims 1-40, specifically binding to CLDN18.2 and not substantially binding to CLDN18.1.

42. The isolated antigen-binding protein of any one of claims 40-41, wherein said CLDN18.2 comprises mouse CLDN18.2, cynomolgus monkey CLDN18.2, and/or human CLDN18.2.

43. The isolated antigen-binding protein of any one of claims 1-42, having CDC activity.

44. The isolated antigen-binding protein of any one of claims 1-43, capable of inhibiting tumor growth and/or proliferation of tumor cells.

45. A chimeric antigen receptor, comprising a targeting moiety, wherein said targeting moiety comprises the antigen-binding protein of any one of claims 1-44.

46. The chimeric antigen receptor of claim 45, comprising a costimulatory domain, wherein said costimulatory domain comprises a costimulatory domain derived from one or more proteins selected from the group consisting of CD28, 4-1BB, CD27, CD2, CD7, CD8, OX40, CD226, DR3, SLAM, CDS, ICAM-1, NKG2D, NKG2C, B7-H3, 2B4, FcεRIγ, BTLA, GITR, HVEM, DAP10, DAP12, CD30, CD40, CD40L, TIM1, PD-1, LFA-1, LIGHT, JAML, CD244, CD100, ICOS, a ligand of CD83, CD40, and MyD88.

47. The chimeric antigen receptor of any one of claims 45-46, wherein said costimulatory domain is an intracellular costimulatory signaling domain derived from 4-1BB.

48. The chimeric antigen receptor of any one of claims 46-47, wherein said costimulatory domain comprises an amino acid sequence as set forth in SEQ ID NO: 85.

49. The chimeric antigen receptor of any one of claims 45-48, comprising an intracellular signaling domain, wherein said intracellular signaling domain comprising an intracellular signaling domain derived from one or more proteins selected from the group consisting of CD3ζ, CD3δ, CD3γ, CD3ε, CD79a, CD79b, FcεRIγ, FcεRIβ, FcyRIIa, bovine leukemia virus gp30, Epstein-Barr virus (EBV) LMP2A, simian immunodeficiency virus PBj 14Nef, Kaposi's Sarcoma Associated-Herpesvirus (HSKV), DAP10, DAP-12, and a domain containing at least one ITAM.

50. The chimeric antigen receptor of claim 49, wherein said intracellular signaling domain is a signaling domain derived from CD3ζ.

51. The chimeric antigen receptor of any one of claims 49-50, wherein said intracellular signaling domain comprises an amino acid sequence as set forth in SEQ ID NO: 86.

52. The chimeric antigen receptor of any one of claims 45-51, comprising a transmembrane domain, wherein said transmembrane domain comprises a transmembrane domain derived from one or more proteins selected from the group consisting of: CD8, CD28, 4-1BB, CD4, CD27, CD7, PD-1, TRAC, TRBC, CD3ε, CD3ζ, CTLA-4, LAG-3, CD5, ICOS, OX40, NKG2D, 2B4, CD244, FcεRIγ, BTLA, CD30, GITR, HVEM, DAP10, CD2, NKG2C, LIGHT, DAP12, CD40L, TIM1, CD226, DR3, CD45, CD80, CD86, CD9, CD16, CD22, CD33, CD37, CD64, CD134, CD137, CD154, and SLAM.

53. The chimeric antigen receptor of claim 52, wherein said transmembrane domain is a transmembrane domain derived from CD8.

54. The chimeric antigen receptor of any one of claims 52-53, wherein said transmembrane domain comprises an amino acid sequence as set forth in SEQ ID NO: 84.

55. The chimeric antigen receptor of any one of claims 45-54, comprising a hinge region between the targeting moiety and the transmembrane domain, wherein said hinge region comprises a hinge region derived from one or more proteins selected from the group consisting of CD28, IgG1, IgG4, IgD, 4-1BB, CD4, CD27, CD7, CD8, PD-1, ICOS, OX40, NKG2D, NKG2C, FcεRIγ, BTLA, GITR, DAP10, CD40L, TIM1, CD226, SLAM, CD30, and LIGHT.

56. The chimeric antigen receptor of claim 55, wherein said hinge region is a hinge region derived from CD8.

57. The chimeric antigen receptor of any one of claims 55-56, wherein said hinge region comprises an amino acid sequence as set forth in SEQ ID NO: 83.

58. The chimeric antigen receptor of any one of claims 45-57, further comprising a signal peptide.

59. The chimeric antigen receptor of claim 58, wherein said signal peptide is a signal peptide derived from the CD8 protein.

60. The chimeric antigen receptor of any one of claims 58-59, wherein said signal peptide comprises an amino acid sequence as set forth in SEQ ID NO: 88.

61. The chimeric antigen receptor of any one of claims 45-60, further comprising a low-density lipoprotein receptor-related protein or a fragment thereof.

62. The chimeric antigen receptor of claim 61, wherein said low-density lipoprotein receptor-related protein or the fragment thereof comprises one or more groups selected from: low-density lipoprotein receptor-related proteins 1-12 and functional fragments thereof.

63. The chimeric antigen receptor of any one of claims 61-62, wherein said low-density lipoprotein receptor-related protein or the fragment thereof comprises an amino acid sequence as set forth in SEQ ID NO: 91.

64. A polypeptide molecule comprising the isolated antigen-binding protein of any one of claims 1-44 or the chimeric antigen receptor of any one of claims 45-63.

65. The polypeptide molecule of claim 64, comprising a fusion protein.

66. An immunoconjugate, comprising the isolated antigen-binding protein of any one of claims 1-46.

67. One or more isolated nucleic acid molecules, encoding the isolated antigen-binding protein of any one of claims 1-44, the chimeric antigen receptor of any one of claims 45-63 or the polypeptide molecule of any one of claims 64-65.

68. The nucleic acid molecule of claim 67, comprising a nucleotide sequence as set forth in any one of SEQ ID NO: 26, SEQ ID NO: 27, SEQ ID NO: 28, SEQ ID NO: 29, SEQ ID NO: 30, SEQ ID NO: 31, SEQ ID NO: 32, and SEQ ID NO: 33.

69. The nucleic acid molecule of any of claims 67-68, comprising any set of nucleotide sequences selected from the groups consisting of:
1) a nucleotide sequence as set forth in SEQ ID NO: 26 and a nucleotide sequence as set forth in SEQ ID NO: 27;
2) a nucleotide sequence as set forth in SEQ ID NO: 30 and a nucleotide sequence as set forth in SEQ ID NO: 27;
3) a nucleotide sequence as set forth in SEQ ID NO: 28 and a nucleotide sequence as set forth in SEQ ID NO: 29;
4) a nucleotide sequence as set forth in SEQ ID NO: 31 and a nucleotide sequence as set forth in SEQ ID NO: 32; and
5) a nucleotide sequence as set forth in SEQ ID NO: 31 and a nucleotide sequence as set forth in SEQ ID NO: 33.

70. A vector, comprising the nucleic acid molecule of any one of claims 67-69.

71. A cell, comprising the isolated antigen-binding protein of any one of claims 1-44, the chimeric antigen receptor of any one of claims 45-63, the polypeptide molecule of any one of claims 64-65, the nucleic acid molecule of any one of claims 67-69, or the vector of claim 70.

72. The cell of claim 71, which is an immune effector cell.

73. The cell of any one of claims 71-72, comprising a T cell, a B cell, a natural killer cell (NK cell), a macrophage, an NKT cell, a monocyte, a dendritic cell, a granulocyte, a lymphocyte, a leukocyte, a peripheral blood mononuclear cell, an embryonic stem cell, a lymphoid progenitor cell, and/or a pluripotent stem cell.

74. The cell of any one of claims 71-73, which is a T cell.

75. The cell of any one of claims 71-74, further comprising and/or expressing a low-density lipoprotein receptor-related protein or a fragment thereof.

76. The cell of claim 75, wherein said low-density lipoprotein receptor-related protein or the fragment thereof comprises one or more groups selected from: low-density lipoprotein receptor-related proteins 1-12 and functional fragments thereof.

77. The cell of any one of claims 75-76, wherein said low-density lipoprotein receptor-related protein or the fragment thereof is low-density lipoprotein receptor-related protein 5 and/or 6 or a fragment thereof.

78. The cell of any one of claims 75-77, wherein said low-density lipoprotein receptor-related protein or the fragment thereof comprises an amino acid sequence as set forth in SEQ ID NO: 91.

79. A pharmaceutical composition, comprising the isolated antigen-binding protein of any one of claims 1-44, the chimeric antigen receptor of any one of claims 45-63, the polypeptide molecule of any one of claims 64-65, the immunoconjugate of claim 66, the nucleic acid molecule of any one of claims 67-69, the vector of claim 70, and/or the cell of any one of claims 71-78, and optionally a pharmaceutically acceptable carrier.

80. A method for preparing the isolated antigen-binding protein of any one of claims 1-44, said method comprising: culturing the cell of claim 71, under the condition that the antigen-binding protein is expressed.

81. A use of the isolated antigen-binding protein of any one of claims 1-44, the chimeric antigen receptor of any one of claims 45-63, the polypeptide molecule of any one of claims 64-65, the immunoconjugate of claim 66, the nucleic acid molecule of any one of claims 67-69, the vector of claim 70, the cell of any one of claims 71-78, and/or the pharmaceutical composition of claim 79, in the manufacture of a medicament, wherein said medicament is used for the prevention, alleviation and/or treatment of a disease and/or a disorder.

82. The use of claim 81, wherein said disease and/or the disorder comprises cancers.

83. The use of any one of claims 81-82, wherein said cancers comprise solid tumors and/or hematomas.

84. The use of any one of claims 82-83, wherein said cancers comprise gastric cancer and/or colon cancer.

85. A method for detecting CLDN18.2 in a sample, said method comprising: administering the isolated antigen-binding protein of any one of claims 1-44, the chimeric antigen receptor of any one of claims 45-63, the polypeptide molecule of any one of claims 64-65, the immunoconjugate of claim 66, the nucleic acid molecule of any one of claims 67-69, the vector of claim 70, the cell of any one of claims 71-78, and/or the pharmaceutical composition of claim 79.

86. A reagent or a kit for detecting CLDN18.2 in a sample, comprising the isolated antigen-binding protein of any one of claims 1-44, the chimeric antigen receptor of any one of claims 45-63, the polypeptide molecule of any one of claims 64-65, the immunoconjugate of claim 66, the nucleic acid molecule of any one of claims 67-69, the vector of claim 70, the cell of any one of claims 71-78, and/or the pharmaceutical composition of claim 79.

87. A use of the isolated antigen-binding protein of any one of claims 1-44, the chimeric antigen receptor of any one of claims 45-63, the polypeptide molecule of any one of claims 64-65, the immunoconjugate of claim 66, the nucleic acid molecule of any one of claims 67-69, the vector of claim 70, the cell of any one of claims 71-78, and/or the pharmaceutical composition of claim 79 in preparation of a kit, wherein said kit is used for detecting the presence and/or content of CLDN18.2 in a sample.
